# EUROPEAN PATENT SPECIFICATION

(11) **EP 1 242 363 B1**
(45) Date of publication and mention of the grant of the patent: **02.11.2005**
(21) Application number: 00985603.0
(22) Date of filing: 18.12.2000
(51) Int. Cl.: C07C 233/76, C07C 233/33, C07D 307/80, C07D 333/22, A61K 31/165, A61K 31/34, A61K 31/38, A61P 37/06

(54) **CD45 INHIBITORS**
CD45 INHIBITOREN
INHIBITEURS DE CD45

(30) Priority: 21.12.1999 US 172788 P
(43) Date of publication of application: 25.09.2002
(73) Proprietor: AstraZeneca AB, 151 85 Södertälje (SE)
(72) Inventor: CHAPDELAINE, Marc, Jerome, Wilmington, DE 19850-5437 (US); KNAPPENBERGER, Katherine, Wilmington, DE 19850-5437 (US); STEELMAN, Gary, Wilmington, DE 19850-5437 (US); SUCHARD, Suzanne, Wilmington, DE 19850-5437 (US); SYGOWSKI, Linda, Wilmington, DE 19850-5437 (US); URBANEK, Rebecca, Wilmington, DE 19850-5437 (US); VEALE, Chris, Allan, Wilmington, DE 19850-5437 (US)
(74) Representative: Bryant, Tracey
(86) International application number: PCT/GB2000/004854
(87) International publication number: WO 2001/046125

(56) References cited:
- US-A- 3 243 444
- US-A- 4 668 712
- US-A- 4 746 678
- US-A- 5 665 774
- CHEMICAL ABSTRACTS, vol. 51, no. 14, 25 July 1957 (1957-07-25) Columbus, Ohio, US; abstract no. 10466c, SHIMPACHIRO KATO ET AL: "Synthesis of bromophenanthrene quinones" XP002162699 & YUKI GOSEI KAGAKU KYOKAI SHI, vol. 15, 1957, pages 84-7,
- CHEMICAL ABSTRACTS, vol. 70, no. 1, 6 January 1969 (1969-01-06) Columbus, Ohio, US; abstract no. 3967b, GORELIK, M V: "Ring opening of 1,2,5-thiazoles and 1,2,5-selenadiazoles under the influence of alkali" page 371; column 2; XP002162700 & ZH. VSES. KHIM. OBSHCHEST., vol. 13, no. 4, 1968, pages 467-8,

## Description

### Background

### Field of the Invention

Compounds, compositions and methods for the treatment of immunologically-related diseases and disorders such as autoimmune disorders and organ graft rejection.

### Related Art

Action of the immune system is known to be involved in immunologically-related diseases and disorders such as autoimmune disorders and in organ graft rejection ("OGR"). Hematopoietic, thymus-derived cells, (so-called "T cells") have an important and pervasive role as regulators and effectors of the functions of the immune system. Hematopoietic cells, and T cells in particular have on their surfaces a major transmembrane glycoprotein designated CD45, characterized by a cluster of antigenic determinants. CD45 is also known as leukocyte common antigen ("LCA"). The cytosolic portion of CD45 has protein tyrosine phosphatase ("PTP") activity and CD45 activity is known to be essential for TCR initiated T cell activation. Studies in CD45-deficient cell lines have shown that CD45 is a positive regulator of the T-Cell Receptor ("TCR") and that CD45 functions in TCR regulation by dephosphorylating the src kinases p56^{*lck*} and p59^{*fyn*}, which allows autophosphorylation of the positive regulatory site on these enzymes: these reactions lead to downstream events and ultimately to T cell activation.

Available treatments for autoimmune disorders and OGR have therapeutic disadvantages. For example, Cyclosporin A, the drug most commonly used to treat OGR, has renal and CNS toxicity.

### Summary of the Invention

Potent inhibitors of CD45 have been discovered. Such inhibitors are useful for the treatment of various autoimmune disorders as well as for treatment of OGR. Inhibition of the phosphatase activity of CD45 by compounds of the present invention has been shown by incubating the cytosolic portion of CD45 with the compounds and *p*-nitrophenyl phosphate (*p*NPP), a phosphatase substrate. Spectrophotometric monitoring has shown that the liberation of *p*-nitrophenol from the substrate by CD45 is inhibited in the presence of the compounds disclosed herein. Inhibition of the phosphatase activity of CD45 by compounds of the present invention has also been shown using a p56^{*lck*} carboxy-terminal phosphorylated peptide as a substrate. Compounds of the present invention have also been shown to inhibit proliferation of T cells in a T-cell proliferation assay.

Compounds of the present invention are substituted phenanthrene-9,10-diones in accord with structural diagram I: 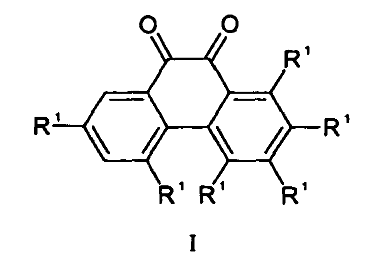 wherein R¹ at each occurrence is independently selected from hydrogen, NH-CO-R², CO-NH-R², Ar, (CH₂)ₙCH(COOH)R³ COR³, NHCOCH₂CH(COOH)NHR⁴ and N(R⁵)₂;
in compounds in accord with structural diagram I, R² is selected from (C₁-C₄)alkyl, (CH₂)ₙCOOR⁶ and phenyl, wherein: n is an integer selected from the range I to 8; phenyl moieties of R² can be substituted at one, two or three positions with a moiety selected from halogen, NO₂ and CF₃; alkyl moieties of R² can be substituted with halogen, and R⁶ is selected from hydrogen and (C₁-C₄)alkyl;
in compounds in accord with structural diagram I where R¹ is Ar, Ar at each occurrence is independently selected from groups in accord with the following structural diagrams: 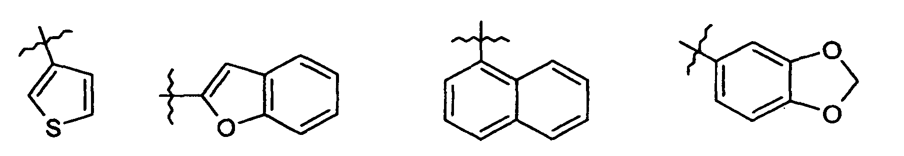 or from phenyl which can be substituted with a moiety selected from halogen, (C₁-C₄)alkyl, O-(C₁-C₄)alkyl and halo(C₁-C₄)alkyl;
in compounds in accord with structural diagram I where R¹ is COR³ or (CH₂)ₙCH(COOH)R³, R³ at each occurrence is an N-linked oligopeptide selected from GQ-N-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPHG, GRPEG, ERPEG, RRPEG, GGPRG, EGPRG, RGPRC GEPRG, EEPRG, REPRG, GRPRG, ERPRG and RRPRG;
in compounds in accord with structural diagram I where R¹ is NHCOCH₂CH(COOH)NHR⁴, R⁴ at each occurrence is a C-linked N-acetyl-amino acid, -tripeptide or -oligopeptide selected from Q, EGQ, ATEGQ, TATEGQ, and FTATEGQ;
in compounds in accord with structural diagram I where R¹ is N(R⁵)₂, R⁵ at each occurrence is selected from hydrogen and tosyl.

Particular compounds of the present invention within the scope of compounds in accord with structural diagram I are substituted phenanthrene-9,10-diones in accord with structural diagram II: 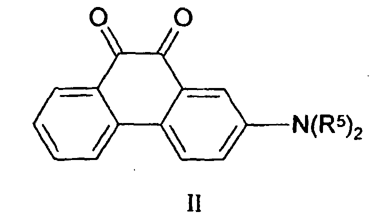 wherein R⁵ is selected from hydrogen and tosyl.

Further particular compounds of the present invention within the scope of compounds in accord with structural diagram I are substituted phenanthrene-9,10-diones in accord with structural diagram III: 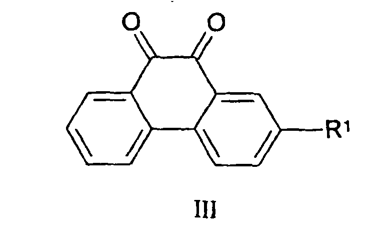 wherein R¹ is Ar and Ar is selected from groups in accord with the following structural diagrams: 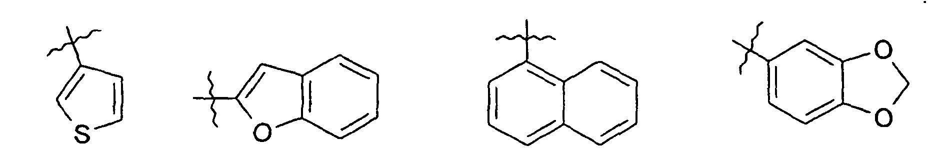 or from phenyl which can be substituted with a moiety selected from halogen, (C₁-C₄)alkyl, halo(C₁-C₄)alkyl and O-(C₁-C₄)alkyl.

Yet further particular compounds of the present invention within the scope of compounds in accord with structural diagram I are substituted phenanthrene-9,10-diones in accord with structural diagram IV: 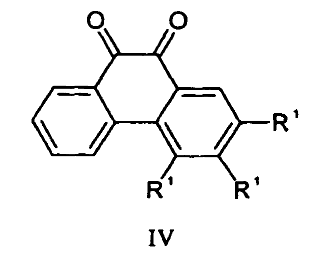 wherein R¹ is independently selected at each occurrence from hydrogen, (CH₂)ₙCH(COOH)R³ and COR³ where R³ is an N-linked peptide selected from GQ-*N*-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPEG, GRPEG, ERPEG, RRPEG, GGPRG, EGPRG, RGPRG, GEPRG, EEPRG, REPRG, GRPRG, ERPRG and RRPRG, with the proviso that no more than one R¹ moiety is other than hydrogen.

Other particular compounds of the present invention within the scope of compounds in accord with structural diagram I are substituted phenanthrene-9,10-diones in accord with structural diagram V: 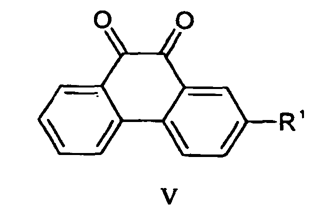 wherein R¹ is NHCOCH₂CH(COOH)NHR⁴, where R⁴ is a C-linked N-acetyl-oligopeptide selected from Q, EGQ, ATEGQ, TATEGQ, and FTATEGQ.

Still further particular compounds of the present invention within the scope of compounds in accord with structural diagram I are phenanthrene-9,10-diones which are not substituted at positions 6 and 8 and wherein R¹ is selected from hydrogen and NHC(O)(CH₂)ₙCOOCH₃ where n is an integer selected from the range 1 to 8.

Compounds of the present invention are ligands of CD45 which, when bound, inhibit the activity of the protein tyrosine phosphatase (PTP) activity of the cytosolic portion of CD45. Binding of a compound of the present invention to CD45 inhibits the activity of CD45 essential for TCR initiated T cell activation. Thus, compounds of the invention inhibit the positive regulation of the TCR that leads to downstream events and T cell activation. Compounds of the present invention are useful to suppress the action of the immune system in immunologically-related diseases and disorders such as autoimmune disorders and organ graft rejection and to inhibit the action of T cells as functional regulators and effectors of the immune system.

The present invention also encompasses compositions made with compounds described herein useful for the treatment of immunologically-related diseases and disorders and methods utilizing such compositions for treating such disorders.

### Detailed Description of the Invention

As described herein, the ring atoms of 9,10-phenanthrenediones are identified with a conventional numbering system, in accord with the following structural diagram:

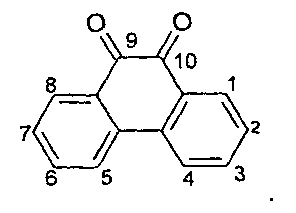

As described herein, amino acids ("AA") of oligopeptides are identified by conventional abbreviations or one-letter code, as follows: Glycine/Gly/G; Glutamic acid/Glu/E; Glutamine/Gln/Q; Proline/Pro/P; Leucine/Leu/L; Arginine/Arg/R; Phenylalanine/Phe/F; Tyrosine/Tyr/Y, and Threonine/Thr/T.

As used herein, (C₁-C₄)alkyl has its conventionally-understood meaning and particularly means linear or branched hydrocarbon chains having from one to four carbon atoms and thus includes methyl, ethyl, propyl, isopropyl, n-butyl, sec-butyl, isobutyl, tert-butyl, and the like.

As used herein, halo(C₁-C₄)alkyl has its conventionally-understood meaning and particularly means (C₁-C₄)alkyl as used herein wherein hydrogen atoms have been replaced by halogen atoms and thus includes monochloromethyl, trifluoromethyl, difluoroethyl, trifluoropropyl, perfluoro(C₁-C₄)alkyl, and the like.

As used herein, perfluoro(C₁-C₄)alkyl has its conventionally-understood meaning and particularly means (C₁-C₄)alkyl as used herein wherein each hydrogen atom has been replaced by a fluorine atom and thus includes trifluoromethyl.

As used herein, (CH₂)ₙ has its conventionally-understood meaning and particularly means linear hydrocarbon chains having from one to n carbon atoms and thus includes methylene, ethylene, propylene, n-butylene groups, and the like.

As used herein, the terms halogen, halo, or halide have their conventionally-understood meanings and particularly mean chlorine, bromine, iodine or fluorine.

As used herein, the term tosyl has its conventionally-understood meaning and particularly means a group in accord with the following structural diagram:

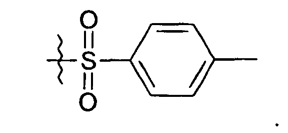

As used herein, the term "from the range 1 to 6" or the like, means any integral value in the stated range, in this example 1, 2, 3, 4, 5 or 6.

### Definitions of terms:

DMF, *N,N*-dimethylformamide; THF, tetrahydrofuran; HATU, *O*-(7-Azabenzotriazol-1-yl)-*N,N,N*'*,N*'-tetramethyluroniumhexafluorophosphate; TLC, thin-layer chromatography; NMR, nuclear magnetic resonance; TFA, trifluoroacetic acid; FMOC, *N*-(9-fluorenylmethoxycarbonyl); HPLC, high performance liquid chromatography; HRMS, high resolution mass spectroscopy; EDC, 1-(3-dimethylaminopropyl)-3-ethylcarbodiimide hydrochloride; DMAP, 4-dimethylaminopyridine; DMSO, dimethylsulfoxide; QTOF, quadropole time of flight; IC₅₀, concentration giving 50% inhibition; CC₅₀, concentration giving 50% cytotoxicity; AA, amino acid; ND, not determined.

HPLC method used: Analytical HPLC using an HP 1100 HPLC. with a C₁₈ Dynamax column (5 cm x 4.6 mm, 3 µM particle size, 100 Å pore size), flow rate of 0.5 mL/min, 20%-60% CH₃CN in H₂O over 7.5 min, holding at 60% CH₃CN for 2.5 min, while monitoring at 254 and 210 nm.

HRMS method used: The sample was solubilized and diluted in MeOH. The QTOF mass spectrometer was calibrated using a PEG 400/600/1000 mixture solubilized in 50:50 acetonitrile/water with 50 mM ammonium acetate across a mass range 150 - 1150 Da. There was one sample submitted, with MW 1262 Da. This sample was run scanning the mass range 500-1300 Da using a calibration from the MW range 150 - 1300. The resolution for the instrument was measured at m/Δm = 5100 at 50% peak height for the ion observed at m/z 953.1443. For each sample, 8 µL of solution was flow-injected into the mass spectrometer with a 35 µL/min flow of 80:20 acetonitrile/water (0.1 % formic acid). A lock mass reference compound was infused into this flow at a rate of 2 µL/min (Ref. Compound exact mass 953.1443).
Data was acquired from a single 3.5 minute analysis, and the signal was averaged from 0.9 - 1.8 min and then smoothed and centroided using a one-point lock mass correction using the reference compound listed above (m/z = 953.1443).

### Examples

### Example 1: N-(9,10-Dioxo-9,10-dihydro-phenanthren-2-yl)-2-fluoro-benzamide:

To a solution of 2-amino-phenanthrene-9,10-dione (50 mg, 240 µmol) in THF (10 mL) was added an excess of Na₂CO₃ (1g), followed by 2-fluorobenzoylchloride (46 µL, 384 µmol). The mixture was shaken overnight, then filtered and the solvent evaporated. The resulting material was purified on a silica gel column, using CH₂Cl₂-10% EtOAC/CH₂Cl₂ as the eluant to yield the pure amide *N*-(9,10-dioxo-9,10-dihydro-phenanthren-2-yl)-2-fluoro-benzamide as a red solid. ¹H NMR (300 MHz, DMSO-d₆) δ 10.80 (1H, s), 8.45 (1H, d, *J* = 2.4 Hz), 8.30 (1H, d, *J =* 9 Hz), 8.23 (1H, d, *J =* 8 Hz), 8.09 (1H, dd, *J =* 2.3, 8.9 Hz), 8.02 (1H, dd, *J* = 1.3, 7.7 Hz), 7.80-7.70 (2H, m), 7.62 (1H, m), 7.50 (1H, dd, *J* = 7.3, 7.3 Hz), 7.38 (2H, m); HPLC: 6.97 min.

The compounds of examples 2 to 8 inclusive were prepared by the method of Example 1, by utilizing the appropriate acid chloride.

### Example 2: 2-Benzyloxy-N-(9,10-dioxo-9,10-dihydro-phenanthren-2-yl)-acetamide:

Mauve solid; ¹H NMR (300 MHz, DMSO-d₆) δ 10.23 (1H, s), 8.38 (1H, d, *J* = 2.1 Hz), 8.26 (1H, d, *J* = 9 Hz), 8.21 (1H, d, *J* =7.8 Hz), 8.05-7.99 (2H, m), 7.76 (1H, dd, *J* = 7.2, 7.2 Hz), 7.51-7.32 (6H, m), 4.65 (2H, s), 4.15 (2H, s); HPLC: 7.51 min.

### Example 3: N-(9,10-Dioxo-9,10-dihydro-phenanthren-2-yl)-butyramide:

Red solid; ¹H NMR (300 MHz, DMSO-d₆) δ 10.25 (1H, s), 8.30 (1H, d, *J* = 2.4 Hz), 8.23 (1H, d, *J* = 8.4 Hz), 8.19 (1 H, d, *J* = 7.2 Hz), 7.98 (2H, m), 7.75 (1H, ddd, *J* = 7.9, 7.9, 1.3 Hz), 7.47 (1H, dd, *J* = 7.2 Hz), 2.34 (2H, t, *J* = 7.2 Hz), 1.64 (2H, tq, *J* = 7.4, 7.4 Hz), 0.93 (3H, t, *J* = 7.3 Hz); HPLC: 5.86 min.

### Example 4: N-(9,10-Dioxo-9,10-dihydro-phenanthren-2-yl)-2,2-dimethylpropionamide:

Mauve solid; ¹H NMR (300 MHz, CDCl₃) δ 8.41 (1H, dd, *J* = 2.5, 8.7 Hz), 8.17 (1H, dd, *J* = 1.3, 7.8 Hz), 7.98 (1H, d, *J* = 8.6 Hz), 7.94 (1H, d, *J* = 7.2 Hz), 7.89 (1H, d, *J* = 2.5 Hz), 7.70 (1H, ddd, *J* = 1.4, 8, 8 Hz), 7.54 (1H, br s), 7.44 (1H, dd, *J* = 7.4, 7.4 Hz), 1.35 (9H, s); HPLC: 6.68 min.

### Example 5: N-(9,10-Dioxo-9,10-dihydro-phenanthren-2-yl)-2-nitro-benzamide:

Purple solid; ¹H NMR (300 MHz, DMSO-d₆) δ 11.04 (1H, s), 8.39 (1H, d, *J* = 2.4 Hz), 8.31 (1H, d, *J =* 9 Hz), 8.24 (1H, d, *J* = 8.1 Hz), 8.19 (1H, d, *J* = 8.1 Hz), 8.04-7.99 (2H, m), 7.86-7.75 (4H, m), 7.50 (1H, dd, *J* = 7.5, 7.5 Hz); HPLC: 6.39 min.

### Example 6: 2,4,6-Trichloro-N-(9,10-dioxo-9,10-dihydro-phenanthren-2-yl)-benzamide:

Red solid; ¹H NMR (300 MHz, ~1:3 DMSO-d₆:CDCl₃) δ 10.89 (1H, s), 8.38 (1H, d, *J =* 1.8 Hz), 8.26 (1H, dd, *J* = 2.4, 8.7 Hz), 8.10 (1H, dd, *J =* 1.1, 7.6 Hz), 8.07 (1H, d, *J =* 8.7 Hz), 8.05 (1H, d, *J* = 7.8 Hz), 7.75 (1 H. ddd, *J* = 1.6, 7.9, 7.9 Hz), 7.47 (3H, m); HPLC: 8.47 min.

### Example 7: 2,2,2-Trichloro-N-(9,10-dioxo-9,10-dihydro-phenanthren-2-yl)-acetamide:

Brown solid; ¹H NMR (300 MHz, CDCl₃) δ 10.61 (1H, br s), 8.43 (1H, d, *J* = 2.1 Hz), 8.35 (1 H, dd, *J* = 2.2, 9 Hz), 8.16 (1H, *dd, J =* 1.2, 7.8 Hz), 8.07-8.01 (2H, m), 7.72 (1H, dd, *J =* 7.5, 7.5 Hz), 7.47 (1H, dd, *J* = 7.7, 7.7 Hz); HPLC: 7.65 min.

### Example 8: N-(9,10-Dioxo-9,10-dihydro-phenanthren-2-yl)-2-trifluoromethylbenzamide:

Brown solid; ¹H NMR (300 MHz, -1:3 DMSO-d₆:CDCl₃) δ 10.78 (1H, s), 8.43 (1H, d, *J* = 2.2 Hz). 8.20 (1 H, dd, *J* = 2.5, 8.8 Hz). 8.10-8.06 (2H, m), 7.84-7.65 (6H, m), 7.46 (1H, dd, *J* = 7.6, 7.6 Hz); HPLC: 7.37 min.

### Example 9: N-(9,10-Dioxo-9,10-dihydro-phenenathren-3-yl)-butyramide:

To a solution of 3-amino-phenanthrene-9,10-dione (50 mg. 240 µmol) in THF (10 mL) an excess of Na₂CO₃ (0.5 g) was added, followed by butyrylchloride (1 mL). The mixture was shaken overnight, then water (1 mL) was added and the mixture was shaken for an additional 30 min. The mixture was filtered through a pad of silica gel using THF as the eluant, and the eluate evaporated under reduced pressure to yield a solid. The solid products were triturated with Et₂O, and the residual solid material collected by vacuum filtration to yield the pure amide *N*-(9,10-dioxo-9,10-dihydro-phenanthren-3-yl)-butyramide as a brown solid. ¹H NMR (300 MHz, DMSO-d₆) δ 10.44 (1H, s), 8.56 (1H, d, *J* = 1.8 Hz), 8.05-8.02 (3H, m), 7.84 (1H, ddd, *J* = 1.5, 7.5, 7.5 Hz), 7.70 (1H, dd, *J* = 1.8, 8.7 Hz), 7.56 (1H, dd. *J* = 7.5, 7.5 Hz), 2.40 (2H, t, *J* = 7.2 Hz), 1.66 (2H, tq, *J* = 7.5, 7.5 Hz), 0.95 (3H, t, *J* = 7.5 Hz); HPLC: 5.97 min.

The compounds of examples 10 to 13 inclusive were prepared by the method of Example 9, by utilizing the appropriate acid chloride.

### Example 10: N-(9,10-Dioxo-9,10-dihydro-phenenathren-3-yl)-2,2-dimethylpropionamide:

Brown solid; ¹H NMR (300 MHz, DMSO-d₆) δ 9.68 (1H, s), 8.60 (1H, s), 8.06-8.02 (3H, m), 7.90 (1H, dd, *J* = 1.8, 8.7 Hz), 7.84 (1H, ddd, *J =* 1.2, 8.1, 8.1 Hz), 7.76 (1H, dd, *J* = 7.5, 7.5 Hz), 1.29 (9H, s); HPLC: 6.89 min.

### Example 11: 2,2,2-Trichloro-N-(9,10-dioxo-9,10-dihydro-phenanthren-3-yl)-acetamide:

Brown solid; ¹H NMR (300 MHz, tfa shake-DMSO-d₆) δ 8.62 (1H, d, J = 1.9 Hz), 8.31 (1H, s), 8.11 (2H, m), 7.96 (1H, dd, *J* = 1.8, 8.7 Hz), 7.86 (1H, ddd, J = 1.5, 7.8, 7.8 Hz); HPLC: 7.76 min.

### Example 12: N-(9,10-Dioxo-9,10-dihydro-phenanthren-3-yl)-2-fluoro-benzamide:

Yellow solid; ¹H NMR (300 MHz, DMSO-d₆) δ 10.91 (1H, s), 8.64 (1H, s), 8.09-8.02 (3H, m), 7.89-7.82 (2H, m), 7.76 (1 H, dd, *J* = 7.5, 7.5 Hz), 7.68-7.57 (2H, m), 7.44-7.36 (2H, m); HPLC: 7.15 min.

### Example 13: N-(9,10-Dioxo-9,10-dihydro-phenanthren-3-yl)-2-trifluoromethylbenzamide:

Brown solid; ¹H NMR (300 MHz, DMSO-d₆) δ 11.10 (1H, s), 8.61 (1H, s), 8.09-7.99 (3H, m), 7.91-7.75 (6H, m), 7.57 (1H, dd, *J* = 7.5, 7.5 Hz); HPLC: 7.36 min.

### Example 14: N-(9,10-Dioxo-9,10-dihydro-phenanthren-2-yl)-4-methyl-N-[(4-methylphenyl)sulfonyl]-benzenesulfonamide:

To a solution of 2-amino-phenanthrene-9, 10-dione (200 mg, 900 µmol) in CH₂Cl₂ (10 mL) under N₂ was added Et₃N (630 µL, 4.48 mmol), *p*-toluenesulfonyl chloride (TsCl, 340 mg, 1.79 mmol) and a catalytic amount of *N,N*-dimethylaminopyridine. The resultant solution was stirred overnight, after which time it was diluted with ethyl acetate (25 mL) and washed sequentially with saturated aqueous NH₄Cl, water and brine and then dried over Na₂SO₄. Filtration followed by evaporation under reduced pressure yielded a product which was purified by silica gel chromatography using CH₂Cl₂ as the eluant; the first material eluted from the column was the di-tosylate of 2-amino-phenanthrene-9,10-dione, *N*-(9,10-dioxo-9,10-dihydro-phenanthren-2-yl)-4-methyl-N-[(4-methylphenyl)sulfonyl]-benzenesulfonamide (39 mg, 73 µmol, 8 %) which was dried to a yellow solid. ¹H NMR (300 MHz, DMSO-d₆) δ 8.39 (1H, d, *J* = 8.7 Hz), 8.31 (1H, d, *J* = 8.0 Hz), 8.06 (1H, dd, *J =* 1.2, 7.7 Hz), 7.81 (1H, dd, *J =* 7.5, 7.5 Hz), 7.73 (4H, d, *J =* 8.3 Hz), 7.60 (1H, *dd, J =* 7.5, 7.5 Hz), 7.54 (1 H, m), 7.52 (4H, *d, J* = 8.3 Hz), 7.36 (1H, dd, *J =* 2.4, 8.5 Hz), 2.50 (6H, s); HPLC: 9.85 min.

### Example 15: N-(9,10-Dioxo-9,10-dihydro-phenanthren-2-yl)-4-methylbenzenesulfonamide:

A second material which eluted from the column was the mono-tosylate of 2-amino-9.10-phenenthrenedione, *N*-(9,10-dioxo-9,10-dihydro-phenanthren-2-yl)-4-methylbenzenesulfonamide (53 mg, 140 mmol, 16%) which was dried to an orange solid. ¹H NMR (300 MHz, DMSO-d₆) δ 10.77 (1H, s), 8.17 (1H, d, *J* = 9 Hz), 8.12 (1H, d, *J* = 8.1 Hz), 7.96 (1H, dd, *J =* 1.5, 7.8 Hz), 7.78-7.29 (4H, m), 7.48 (1H, s), 7.46 (1H, t, *J* = 8.4 Hz), 7.38 (2H, d, *J* = 8.1 Hz), 2.32 (3H, s); HPLC: 7.14 min.

### Example 16: 2-Benzofuran-2-yl-phenanthrene-9,10-dione:

To a mixture of 2-bromo-phenanthrene-9,10-dione (150 mg, 520 µmol) in dioxane (5 mL) and H₂O (0.5 mL), benzo[b]furan-2-boronic acid (169 mg, 1.4 mmol), *tris*(dibenzylideneacetone)-dipalladium(0) (48 mg, 52 µmol), tri-*o*-tolylphosphine (32 mg, 1.4 mmol) and K₂CO₃ (220 mg, 1.57 mmol) were added. The resultant mixture was heated at 80 °C for 18 h and the solid material was removed by filtration. The soluble material was purified using a Rainin preparative HPLC on a Rainin phenyl column (25 cm x 21.4 mm, 8 µM particle size, 60 Å), 40 mL/min, with 50% - 80% dioxane/H₂O for 40 min, followed by 80% - 50% for 5 min. If the purity at this stage was not sufficient, the material was then chromatographed on a silica gel column using CH₂Cl₂ as the eluant. Purple solid; ¹H NMR (300 MHz, CDCl₃) δ 8.64 (1H, d, *J* = 1.8 Hz), 8.21 (2H, ddd, *J* = 1.2, 7.8, 7.8 Hz), 8.10 (1H, d, *J* = 8.7 Hz), 8.05 (1H, d, *J* = 7.8 Hz), 7.75 (1H, dd, *J* = 7.8, 7.8 Hz), 7.64 (1H, d, *J* = 7.5 Hz), 7.57 (1H, d, *J* = 8.1 Hz), 7.50 (1H, dd, *J* = 7.8, 7.8 Hz), 7.35 (1H, ddd, *J* = 1.2, 6.6, 6.6 Hz), 7.31-7.28 (2H, m); HPLC: 10.97 min.

The compounds of examples 17 to 22 inclusive were prepared by the method of Example 16, by utilizing the appropriate boronic acid.

### Example 17: 2-Thiophen-3-yl-phenanthrene-9,10-dione:

Orange solid; ¹H NMR (300 MHz, CDCl₃) δ 8.40 (1H, d, *J*= 2.1 Hz), 8.20 (1H, d, *J* = 7.8 Hz), 8.04 (1H, d, *J* = 8.1 Hz) 8.02 (1 H, d, *J* = 7.2 Hz), 7.94 (1H, dd, *J* = 2.1, 8.4 Hz), 7.73 (1H, dd, *J* = 7.5, 7.5 Hz), 7.64 (1H, m), 7.51-7.45 (3H, m); HPLC: 8.47 min.

### Example 18: 2-(2-Fluoro-phenyl)-phenanthrene-9,10-dione:

Orange solid; ¹H NMR (300 MHz, CDCl₃) δ 8.38 (1H, s), 8.23 (1H, d, *J* = 7.8 Hz), 8.10 (1H, d, *J* = 8.1 Hz), 8.07 (1H, d, *J* = 8.1 Hz), 7.95 (1H, m), 7.75 (1H, dd, *J* = 7.5, 7.5 Hz), 7.56-7.51 (2H, m), 7.41-7.38 (1H, m), 7.28-7.20 (2H, m); HPLC: 8.76 min.

### Example 19: 1-Naphthalen-2-yl-phenanthrene-9,10-dione:

Orange solid; ¹H NMR (300 MHz, CDCl₃) δ 8.35 (1H, d, *J* = 2.1 Hz), 8.25 (1H, dd, *J* = 1.2, 7.2 Hz), 8.16 (1 H, d, *J* = 8.4 Hz), 8.11 (1H, d, *J* = 8.1 Hz), 7.96-7.84 (4H, m), 7.77 (1H, ddd, *J* = 1.5, 7.7, 7.7 Hz), 7.52-7.47 (5H, m); HPLC: 10.31 min.

### Example 20: 2-(2-Methoxy-phenyl)-phenanthrene-9,10-dione:

Orange solid; ¹H NMR (300 MHz, CDCl₃) δ 8.36 (1H, d, *J* = 1.9 Hz), 8.20 (1H, dd, *J* = 1.4, 7.8 Hz), 8.06 (1 H, s), 8.03 (1H, s), 7.93 (1H, dd, *J* = 1.9, 8.3 Hz), 7.73 (1H, ddd, *J* = 1.5, 7.6, 7.6 Hz), 7.47 (1H, ddd, *J* = 0.6, 7.4, 7.4 Hz), 7.42-7.36 (2H, m), 7.10 (2H, m), 3.86 (3H, s); HPLC: 8.71 min.

### Example 21: 2-Benzo[1,3]dioxol-5-yl-phenanthrene-9,10-dione:

Violet solid; ¹H NMR (300 MHz, CDCl₃) δ 8.33 (1H, d, *J=* 2.1 Hz), 8.20 (1 H, dd, *J* = 1.5, 7.8 Hz), 8.04 (1H, d, J = 8.1 Hz), 8.02 (1 H, d, J = 7.2 Hz), 7.86 (1H, dd, *J* = 1.8, 8.4 Hz), 7.73 (1H, ddd, *J* = 1.5, 7.8, 7.8 Hz), 7.47 (1H, ddd, *J* = 0.9, 7.4, 7.4 Hz), 7.21-7.14 (2H, m), 6.91 (1H, d, *J* = 7.8 Hz), 6.10 (2H, s); HPLC: 8.71 min.

### Example 22: 2-(2-Ethoxy-phenyl)-phenanthrene-9,10-dione:

Orange solid; ¹H NMR (300 MHz, CDCl₃) δ 8.43 (1H, s), 8.21 (1H, d, *J* = 7.8 Hz), 8.04 (2H, m), 7.96 (1H, d, *J* = 8.4 Hz), 7.73 (1H, dd, *J* = 7.2, 7.2 Hz), 7.49-7.34 (3H, m), 7.12-6.99 (2H, m), 4.09 (2H, q, *J* = 7.2 Hz), 1.39 (3H, t, *J* = 6.9 Hz); HPLC: 9.55 min.

### Example 23: A compound in accord with structural diagram VII:

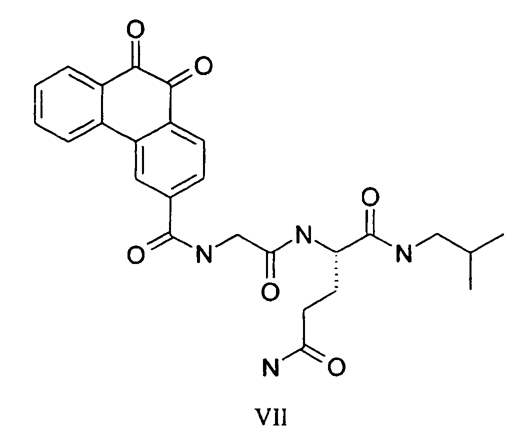

Polystyrene resin with the Ellman's aldehyde linker (50 mmol, see *J. Org. Chem*. **1997**, *62*, p. 1240) was swelled in DMF (1 L) and acetic acid (10 mL) for 10 minutes, at which time isobutylamine (32 mL, 325 mmol) and sodium triacetoxyborohydride (69.5 g, 328 mmol) were added. The mixture was stirred with an overhead stirrer for two hours, then transferred to a fritted glass funnel and was washed with MeOH and DMF (1:1 mixture, 3 x 300 mL), DMF (3 x 300 mL), CH₂Cl₂ (5 x 300 ml) and methanol (5 x 300 ml). The resin was dried *in vacuo* at 40 °C for 16 h. The above resin (1.2 g. 1 mmol) was swelled in DMF for 15 min, then washed three times with DMF. A solution of FMOCGln(Trt)OH (2.14 g, 3.5 mmol), HATU (1.14 g, 3 mmol), and diisopropylethylamine (1.1 mL, 6 mmol) in DMF (10 mL) was added to the resin and allowed to react for one hour. The liquid was drained and the resin was washed with DMF (10 x 10 mL). The resin was treated with 20% piperidine in DMF (10 mL) for 3 min, drained, then again treated with 20% piperidine in DMF (10 mL) for 7 min and drained. The resin was washed with DMF (10 x 10 mL). A mixture of FMOCGlyOH (1.04 g, 3.5 mmol). HATU (1.14 g, 3 mmol), and diisopropylethylamine (1.1 mL, 6 mmol) in DMF (10 mL) was added to the resin and allowed to react for1 h. The liquid was drained and the resin was washed with DMF (10 x 10 mL). The resin was treated with 20% piperidine in DMF (10 mL) for 3 min, drained, then treated with another 20% piperidine in DMF (10 mL) for 7 min and drained. The resin was washed with DMF (10 x 10 mL). A mixture of 9,10-dioxo-9,10-dihydro-phenanthrene-3-carboxylic acid (880 mg, 3.5 mmol), HATU (1.14 g, 3 mmol), and diisopropylethylamine (1.1 mL, 6 mmol) was added to the resin and allowed to react for 1 h. The liquid was drained and the resin washed with DMF (10 x 10 mL), CH₂Cl₂ (5 x 10 mL), and Et₂O (5 x 10 mL). The resin was dried for 16 h *in vacuo*, then treated with TFA containing 2% water and 2% thioanisole (10 mL) for 2 h. The solids were removed by filtration and washed with CH₂Cl₂ (10 mL). The volume of combined filtrate was reduced to about 1 mL by rotary evaporation and precipitation was induced by addition of Et₂O (25 mL). The resulting solid was collected by vacuum filtration, washed with H₂O (3 x 25 mL) and dried *in vacuo* for 2 h. This product was purified by preparative HPLC on a C₁₈ Dynamax column (21.4 mm X 25 cm, 60 A) using a gradient of 10% to 40% acetonitrile in water with 0.1% TFA. Fractions containing pure product were combined and lyophilized to give the compound in accord with structural diagram VII (170 mg) as an orange solid. Orange solid; HPLC: 3.95 min. Anal. Calcd. for C₂₆H₂₈N₄O₆-0.5H₂O-0.5CF₃CO₂H C, 58.06; H, 5.32; N, 10.03. Found: C, 57.98, 58.31; H, 5.48, 5.40; N, 9.88, 9.88.

### Example 24: A compound in accord with structural diagram VIII:

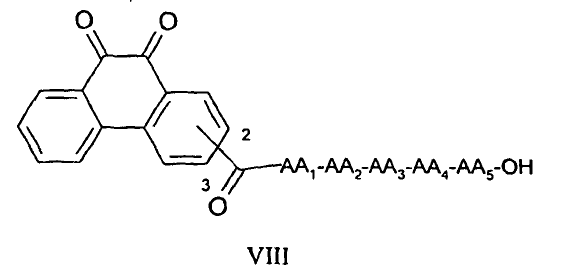

6-Chlorochlortrityl resin (6.19 g, 7 mmol) was swelled in dry CH₂Cl₂ (30 mL) and a solution of FMOCprolineOH (1.18 g, 3.5 mmol) and diisopropylethylamine (1.8 mL, 10.5 mmol) in dry CH₂Cl₂ (10 mL) was added. The reaction was gently mixed under a nitrogen atmosphere for 30 min, then MeOH (3 mL) was added to cap the unreacted sites. After 15 min, the resin was transferred to a fritted glass funnel and washed with DMF (2 x 50 mL), CH₂Cl₂ (2 x 50 mL). MeOH (2 x 50 mL), and Et₂O (2 x 50 mL). The resin was dried *in vacuo* at 50 °C to constant weight. A portion of this material (1.5 g, 0.25 mmol) was swelled in DMF (5 mL) for 15 min then washed with DMF (3 x 5 mL). The resin was treated with 20% piperidine in DMF (5 mL) for 3 min, drained, then treated with another 20 % piperidine in DMF (5 mL) for 7 min and drained. The resin was washed with DMF (10 x 5 mL). A solution of FMOCglutamine(Trt)OH (610 mg, 1 mmol), HATU (380 mg, 1 mmol), and diisopropylethylamine (350 µL) in DMF (5 mL) was added to the resin and mixed with a gentle nitrogen bubbling for 2 h. The solution was drained and the resin was washed with DMF (10 x 5 mL). The resin was treated with 20% piperidine in DMF (5 mL) for 3 min, drained, then another portion of 20% piperidine in DMF (5 mL) was added and mixed for 7 min then drained. The resin was washed with DMF (10 x 5 mL). A solution of FMOCprolineOH (340 mg, I mmol), HATU (380 mg, 1 mmol), and diisopropylethylamine (350 µL) in DMF (5 mL) was added to the resin and mixed with a gentle nitrogen bubbling for 16 h. The solution was drained and the resin was washed with DMF (10 x 5 mL). The resin was treated with 20% piperidine in DMF (5 mL) for 3 min. drained, then another portion of 20% piperidine in DMF (5 mL) for 7 min and drained. The resin was washed with DMF (10 x 5 mL). A solution of FMOCglutamineOH (370 mg, 1 mmol), HATU (380 mg, 1 mmol), and diisopropylethylamine (350 µL) in DMF (5 mL) was added to the resin and mixed with a gentle nitrogen bubbling for 2.5 h. The solution was drained and the resin washed with DMF (10 x 5 mL). The resin was treated with 20% piperidine in DMF (5 mL) for 3 min. drained, then with another 20% piperidine in DMF (5 mL) for 7 min and drained. The resin was washed with DMF (10 x 5 mL). A solution of FMOCglutamateOH(OtBu) (830 mg, 1 mmol), HATU (380 mg. 1 mmol), and diisopropylethylaminc (350 µL) in DMF (5 mL) was added to the resin and mixed with a gentle nitrogen bubbling for 2 h. The solution was drained and the resin was washed with DMF (10 x 5 mL). The resin was treated with 20% piperidine in DMF (5 mL) for 3 min, drained, then with another 20% piperidine in DMF (5 mL) for 7 min and drained. The resin was washed with DMF (10 x 5 mL). A solution of 9,10-dioxo-9,10-dihydro-phenanthrene-3-carboxylic acid (250 mg, I mmol), HATU (380 mg, 1 mmol), and diisopropylethylamine (350 µL) in DMF (5 mL) was added to the resin and mixed with a gentle nitrogen bubbling for 16 h. The solution was drained and the resin was washed with DMF (10 x 5 mL), CH₂Cl₂ (5 x 5 mL), Et₂O (5 x 5mL), then dried under a stream of N₂. The resin was treated with TFA containing 2% thioanisole and 2% water (5 mL) for 3 h. The solids were filtered off and the filtrate was evaporated to a red oil. Addition of Et₂O (10 mL) induced precipitation, and this solid was collected by vacuum filtration to give the product (300 mg) as a pink solid. The material was purified by preparative HPLC on a C₁₈ Dynamax column (21.4 mm X 25 cm, 60 Å) using a gradient of 10% to 26% acetonitrile in water with 0.1% TFA. Fractions containing pure product were combined and lyophilized to give M374187 (100 mg) as a yellow solid. HPLC: 2.55 min; Anal. Calcd. for C₄₀H₄₅N₇O₁₃-1.0H₂O-0.5CF₃CO₂H C, 54.30; H, 5.28; N, 10.81; Found C. 54.10, 54.04; H, 5.19, 5.23; N, 10.99, 11.01.

The compounds shown in tables I and 2, below, were synthesized by the method of Example 24, using either 9,10-dioxo-9,10-dihydro-phenanthrene-3-carboxylic acid or 9,10-dioxo-9,10-dihydro-phenanthrene-2-carboxylic acid as the starting material and a suitable peptide fragment:

Table 1 shows compounds of the invention in accord with structural diagram VIII, having 3-carboxy-linked peptides.

**Table 1:**

| **Example No.** | **AA**_{**1**} | **AA**_{**2**} | **AA**_{**3**} | **AA**_{**4**} | **AA**_{**5**} |
|---|---|---|---|---|---|
| **24** | Glu | Gln | Pro | Gln | Pro |
| **25** | Gly | Gln | Pro | Gln | Pro |
| **26** | Gln | Gln | Pro | Gln | Pro |
| **27** | Gly | Glu | Pro | Gln | Pro |
| **28** | Gln | Glu | Pro | Gln | Pro |
| **29** | Gly | Gin | Gly | Gln | Pro |
| **30** | Gln | Gln | Gly | Gln | Pro |
| **31** | Glu | Gln | Gly | Gln | Pro |
| **32** | Gly | Glu | Gly | Gin | Pro |
| **33** | Gln | Glu | Gly | Gin | Pro |
| **34** | Gln | Gin | Pro | Glu | Gly |
| **35** | Gly | Gln | Gly | Glu | Pro |
| **36** | Gly | Gln | Pro | Gln | Gly |
| **37** | Gln | Gin | Pro | Gln | Gly |
| **38** | Gly | Glu | Pro | Gln | Gly |
| **39** | Gly | Gln | Pro | Glu | Gly |

Example 25: Yellow solid; HPLC: 2.67 min; Anal. Calc. For C₃₇H₄₁N₇O₁₁-1.5H₂O-0.4CF₃CO₂H C, 54.54; H, 5.38; N, 11.78. Found: C, 54.52, 54.42; H, 5.40, 5.32; N, 11.72, 11.80.

Example 26: Yellow solid; HPLC: 2.41 min.

Example 27: Yellow solid; HPLC: 2.88 min; Anal. Calcd. for C₃₇H₄₀N₆O₁₂-1.0H₂O-0.5CF₃CO₂H C, 54.61; H, 5.13; N, 10.05. Found C, 54.82, 54.81; H, 5.51, 5.52; N, 10.01; 10.01.

Example 28: Yellow solid; HPLC: 2.60 min; Anal. Calcd. for C₄₀H₄₅N₇O₁₃-1.5H₂O-0.6CF₃CO₂H C. 53.37; H, 5.28; N, 10.57. Found C, 53.49, 53.43; H, 5.14, 5.15; N, 10.85, 10.82.

Example 29: Yellow solid; HPLC: 2.34 min; Anal. Calcd. for C₃₄H₃₇N₇O₁₁-0.7H₂O-0.6CF₃CO₂H C, 52.80; H, 4.91; N, 12.24. Found C, 52.59, 52.81; H, 4.92, 4.90; N, 12.53, 12.57.

Example 30: Yellow solid; HPLC: 2.07 min; Anal. Calcd. for C₃₇H₄₂N₈O₁₂-2.0H₂O-0.6CF₃CO₂H C, 51.25; H, 5.25; N, 12.52. Found C, 51.31, 51.39; H, 4.97, 4.97; N, 12.75, 12.79.

Example 31: Yellow solid; HPLC: 2.47 min: Anal. Calcd. for C₃₇H₄₁N₇O₁₃-1.0H₂O-0.5CF₃CO₂H C, 52.66; H, 5.06; N, 11.31. Found C. 52.79, 52.78; H, 5.17, 5.20; N, 11.31, 11.31.

Example 32: Yellow solid; HPLC: 2.52 min; Anal. Calcd. for C₃₄H₃₆N₆O₁₂-0.5H₂O-0.5CF₃CO₂H C, 53.44; H, 4.80; N, 10.69. Found 53.29, 53.24; H, 4.96. 4.98; N, 10.73. 10.74.

Example 33: Yellow solid; HPLC: 2.25 min; Anal. Calcd. for C₃₇H₄₁N₇O₁₃-1.0H₂O-0.5CF₃CO₂H C, 52.66; H, 5.06; N, 11.31. Found C, 52.59, 52.61; H, 5.17, 5.18; N, 11.46, 11.37.

Example 34: Yellow solid; HPLC: 2.13 min; Anal. Calcd. for C₃₇H₄₁N₇O₁₃-1.5H₂O-0.5CF₃CO₂H C. 52.11; H, 5.12; N, 11.20. Found C. 51.94, 51.98; H, 4.93, 4.93; N, 11.42, 11.42.

Example 35: Yellow solid; HPLC: 2.49 min; Anal. Calcd. for C₃₄H₃₆N₆O₁₂-1.5H₂O-0.5CF₃CO₂H C. 52.24; H. 4.95; N. 10.44. Found C. 51.87, 51.91; H, 4.93, 4.86; N, 10.56, 10.57.

Example 36: Yellow solid; HPLC: 2.26 min; Anal. Calcd. for C₃₄H₃₇N₇O₁₁-1.0H₂O-0.5CF₃CO₂H C, 52.92, 53.08; H, 5.01, 5.00; N, 12.60, 12.66.

Example 37: Yellow solid; HPLC: 2.02 min; Anal. Calcd. for C₃₇H₄₂N₈O₁₂-1.0H₂O-0.6CF₃CO₂H C, 52.30; H, 5.12; N, 12.77. Found C, 52.35, 52.28; H, 5.21. 5.15; N. 13.08, 13.05.

Example 38: Yellow solid; HPLC: 2.50 min; Anal. Calcd. for C₃₄H₃₆N₆O₁₂-0.5H₂O-0.5CF₃CO₂H C, 53.4; H, 4.80; N. 10.68. Found C. 53.48, 53.48; H. 4.80, 4.85; N, 10.84, 10.88.

Example 39: Yellow solid; HPLC: 2.47 min: Anal. Calcd. for C₃₄H₃₆N₆O₁₂-1.0H₂O-0.5CF₃CO₂H C, 52.83; H. 4.87; N. 10.56. Found C, 52.99, 53.30; H, 4.80, 4.83; N, 10.87, 10.84.

Table 2 shows compounds of the invention in accord with structural diagram VIII, having 2-carboxy-linked peptides.

**Table 2:**

| **Example No.** | **AA**_{**1**} | **AA**_{**2**} | **AA**_{**3**} | **AA**_{**4**} | **AA**_{**5**} |
|---|---|---|---|---|---|
| **40** | Gly | Gly | Pro | Glu | Gly |
| **41** | Glu | Gly | Pro | Glu | Gly |
| **42** | Arg | Gly | Pro | Glu | Gly |
| **43** | Gly | Glu | Pro | Glu | Gly |
| **44** | Glu | Glu | Pro | Glu | Gly |
| **45** | Arg | Glu | Pro | Glu | Gly |
| **46** | Gly | Arg | Pro | Glu | Gly |
| **47** | Glu | Arg | Pro | Glu | Gly |
| **48** | Arg | Arg | Pro | Glu | Gly |
| **49** | Gly | Gly | Pro | Arg | Gly |
| **50** | Glu | Gly | Pro | Arg | Gly |
| **51** | Arg | Gly | Pro | Arg | Gly |
| **52** | Gly | Glu | Pro | Arg | Gly |
| **53** | Glu | Glu | Pro | Arg | Gly |
| **54** | Arg | Glu | Pro | Arg | Gly |
| **55** | Gly | Arg | Pro | Arg | Gly |
| **56** | Glu | Arg | Pro | Arg | Gly |
| **57** | Are | Arg | Pro | Arg | Gly |

Example 40: Yellow solid: HPLC: 2.77 min; Anal. Calc. For C₃₁H₃₁N₅O₁₁-1.0H₂O-0.5CF₃CO₂H C. 53.04; H, 4.66; N, 9.66. Found: C, 53.10, 52.99; H, 4.68, 4.61; N, 9.87, 9.93.

Example 41: Yellow solid; HPLC: 2.83 min; Anal. Calc. For C₃₄H₃₅N₅O₁₃-0.5H₂O-0.5CF₃CO₂H C. 53.37; H, 4.67; N, 8.89. Found: C, 53.35, 53.46; H, 4.75, 4.83; N, 8.85, 8.89 min.

Example 42: Yellow solid; HPLC: 2.74 min; Anal. Calc. For C₃₅H₄₀N₈O₁₁-0.5H₂O-1.5CF₃CO₂H C, 49.14; H, 4.61; N, 12.06. Found: C, 49.07, 49.12; H, 4.64, 4.70; N, 12.06, 12.08.

Example 43: Yellow solid; HPLC: 2.78 min; Anal. Calc. For C₃₄H₃₅N₅O₁₃-0.75H₂O-0.4CF₃CO₂H C, 53.53; H, 4.76; N, 8.97. Found: C, 53.75, 53.75; H, 4.80, 4.79; N, 9.04, 9.06.

Example 44: Yellow solid; HPLC: 2.83 min; Anal. Calc. For C₃₇H₃₉N₅O₁₅-0.5H₂O-0.5CF₃CO₂H C, 53.09; H, 4.75; N, 8.15. Found: C, 52.92, 52.86; H, 4.94, 4.88; N, 8.22, 8.22.

Example 45: Yellow solid; HPLC: 2.74 min; Anal. Calc. For C₃₈H₄₄N₈O₁₃-1.5H₂O-1.4CF₃CO₂H C, 48.64; H, 4.84; N, 11.12. Found: C, 48.36, 48.46; H, 4.68, 4.71; N, 11.23, 11.28.

Example 46: Yellow solid; HPLC: 2.49 min; Anal. Calc. For C₃₅H₄₀N₈O₁₁-0.5H₂O-1.5CF₃CO₂H C, 49.14; H, 4.61; N, 12.06. Found: C, 49.00, 48.67; H, 4.68, 4.67; N, 12.24, 12.14.

Example 47: Yellow solid; HPLC: 2.83 min; Anal. Calc. For C₃₈H₄₄N₈O₁₃-1.0H₂O-1.5CF₃CO₂H C, 48.76; H, 4.74; N, 11.10. Found: C, 48.69, 48.96; H, 4.74, 4.76; N, 11.33, 11.34.

Example 48: Yellow solid; HPLC: 2.48 min; Anal. Calc. For C₃₉H₄₉N₁₁O₁₁-2.0H₂O-2.2CF₃CO₂H C, 45.94; H, 4.90; N, 13.58. Found: C, 45.72, 45.90; H. 4.61, 4.62; N, 13.75, 13.79. C, 45.94; H, 4.90; N, 13.58. Found: C, 45.72, 45.90; H, 4.61, 4.62; N, 13.75, 13.79.

Example 49: Yellow solid; HPLC: 2.74 min; Anal. Calc. For C₃₂H₃₆N₈O₉-0.5H₂O-1.5CF₃CO₂H C, 49.07; H, 4.53; N, 13.08. Found: C, 49.06, 49.10; H, 4.53, 4.54; N, 13.30, 13.29.

Example 50: Yellow solid; HPLC: 2.84 min; Anal. Calc. For C₃₅H₄₀N₈O₁₁-0.5H₂O-1.5CF₃CO₂H C, 49.14; H, 4.61; N, 12.06. Found: C, 48.87, 49.00; H, 4.63, 4.64; N, 12.23, 12.29.

Example 51: Yellow solid; HPLC: 2.49 min; Anal. Calc. For C₃₆H₄₅N₁₁O₉-1.5H₂O-2.5CF₃CO₂H C, 45.27; H, 4.68; N, 14.16. Found: C. 45.10, 45.14; H, 4.60, 4.55; N, 14.27, 14.28.

Example 52: Yellow solid; HPLC: 2.81 min; Anal. Calc. For C₃₅H₄₀N₈O₁₁-1.25H₂O-1.5CF₃CO₂H C, 48.44; H, 4.71; N, 11.89. Found: C, 48.40, 48.28; N, 4.62, 4.60; N, 12.13, 12.10.

Example 53: Yellow solid; HPLC: 2.92 min; Anal. Calc. For C₃₈H₄₄N₈O₁₃-1.0H₂O-1.5CF₃CO₂H C, 48.76; H, 4.74; N, 11.10. Found: C, 48.36, 48.52; H, 4.67, 4.69; N, 11.20. 11.26.

Example 54: Yellow solid; HPLC: 2.56 min; Anal. Calc. For C₃₉H₄₉N₁₁O₁₁-1.5H₂O-2.3CF₃CO₂H C, 46.05; H, 4.81; N. 13.55. Found: C, 45.83, 45.90; H, 4.63, 4.63; N, 13.79, 13.73.

Example 55: Yellow solid; HPLC: 2.57 min; Anal. Calc. For C₃₆H₄₅N₁₁O₉-2.0H₂O-2.2CF₃CO₂H C, 45.66; H, 4.86; N, 14.50. Found: C, 45.46, 45.58; H, 4.57, 4.58; N, 14.57, 14.61.

Example 56: Yellow solid; HPLC: 2.71 min; Anal. Calc. For C₃₉H₄₉N₁₁O₁₁-1.5H₂O-2.3CF₃CO₂H C, 46.05; H, 4.81; N, 13.55. Found: C, 46.03, 45.83; H, 4.68, 4.65; N, 13.74, 13.72.

Example 57: Yellow solid; HPLC: 2.09 min; Anal. Calc. For C₄₀H₅₄N₁₄O₉-2.5H₂O-3.2CF₃CO₂H C, 43.37; H, 4.89; N, 15.26. Found: C, 43.40, 43.28; H, 4.57, 4.52; N, 15.38, 15.34.

Example 58: A compound in accord with structural diagram IX:

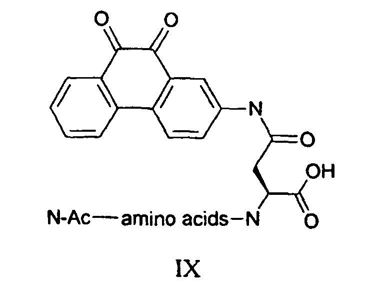

A compound in accord with structural diagram IX having the N-Ac-amino acid sequence N-Ac-Glu-Gly-Gln was synthesized as follows: FMOCAsp(OH)OtBu (3.3 g, 8 mmol) and *N*-methylmorpholine (900 µL, 8 mmol) were dissolved in dry THF (20 mL) and chilled to -10 °C. Isobutylchloroformate (910 µL, 7 mmol) was added dropwise, maintaining reaction temperature below -7 °C. To this mixture was added a suspension of 2-amino-phenanthrene-9,10-dione (1.12 g, 5 mmol) in dry THF (40 mL), and the reaction was allowed to warm to ambient temperature. The disappearance of 2-aminophenanthrene-9,10-dione was monitored by TLC (R_{f} = 0.42, 25:75 EtOAc:CH₂Cl₂, v/v on a silica gel plate), and reaction was complete after 7 d. The solvent was removed by rotary evaporation and the residue was partitioned between EtOAc and sat'd. aq. NH₄Cl. The organic phase was washed with sat'd. aq. NaHCO₃ and brine, then dried over anhydrous MgSO₄. This mixture was filtered and the filtrate was concentrated to a red-brown solid and chromatographed on silica gel with 15:85, EtOAC:CH₂Cl₂, v/v. This gave FMOCAsp-2-aminophenanthrene-9,10-dione (1.92 g, 3.1 mmol, 63%) as a red solid. This intermediate was dissolved in CH₂Cl₂ (100 mL) and TFA (30 mL) was added; after 1h the volatiles were removed by rotary evaporation. Residual TFA was removed by dissolving the material in CH₂Cl₂ and concentration on the rotary evaporator twice. This was dissolved in dry CH₂Cl₂ (100 mL), diisopropylethylamine (5 mL) and dry DMF (10 mL) were added under N₂. 2-chlorochlortrityl resin (4.5 g of 1.2 mmol/g, 5.5 mmol) was added to the mixture and mixed by shaking. After 3 h, MeOH (10 mL) was added and the shaking was continued for an additional 10 min. The resin was collected by vacuum filtration and washed with CH₂Cl₂ (5 x 10 mL), DMF (2 x 10 mL). CH₂Cl₂ (2 x 10 mL) and MeOH (3 x 10 mL). The resin was dried for 64 h *in vacuo*. An incorporation of 2.3 mmol of FMOCAsp(3-aminophenanthrene-9,10-dione) was calculated based on the increase of mass to 5.8 g. The resin was swelled in DMF (25 mL) for 1 h, then washed with DMF (5 x 25 mL). The resin was treated with 20% piperidine in DMF (10 mL) for 3 min, drained, then was treated with more 20% piperidine in DMF (10 mL) for 7 min and drained. The resin was washed with DMF (10 x 10 mL). A solution of FMOCGIn(trt)OH (6.72 g. 11 mmol), HATU (3.8 g, 10 mmol), and diisopropylethylamine (3.5 mL) in DMF (15 mL) was added to the resin and mixed with gentle nitrogen bubbling for 2 h. The solution was drained and the resin was washed with DMF (10 x 10 mL). The resin was treated with 10 ml 20% piperidine in DMF (10 mL) for 3 min, drained, then another portion of 20% piperidine in DMF (10 mL) was added for 7 min and drained. The resin was washed with DMF (10 x 10 mL). A portion of the resin (ca. 10%) was removed for other reactions and the remaining resin was treated with a solution of FMOCGlyOH (1.93 g, 11 mmol). HATU (3.8 g, 10 mol), diisopropylethylamine (3.5 mL) in DMF (15 mL) for 1 h. The reaction was drained and washed with DMF (10 x 10 mL). The resin was treated with 20% piperidine in DMF (10 mL) for 3 min, drained, then treated with additional 20% piperidine in DMF (10 mL) for 7 min and drained. The resin was washed with DMF (10 x 10 mL). A portion of the resin (about 10%) was removed for other reactions and the remaining resin was treated with a solution of FMOCGlu(tBu)OH (4.68 g, 11 mmol), HATU (3.8 g. 10 mmol), and diisopropylethylamine (3.5 mL) in DMF (15 mL) for 16 h. The solution was drained and the resin was washed with DMF (10 x 10 mL). The resin was treated with 20% piperidine in DMF (10 mL) for 3 min, drained, then additional 20% piperidine in DMF (10 mL) was added for 7 min and drained. The resin was washed with DMF (10 x 10 mL). A portion of this material (ca. 15%) was removed and treated with a solution of Ac₂O (1 mL) and diisopropylethylamine (2 mL) in DMF (5 mL) for 2 h. The solution was drained and washed withDMF (10 x 5 mL), CH₂Cl₂ (5 x 5 mL), Et₂O (5 x 5 mL) and dried under a N₂ stream. The resin was treated with a solution of TFA containing 2% H₂O and 2% thioanisole (5 mL) for 2 h. The solids were removed and the solution was concentrated to a red oil. Addition of Et₂O (10 mL) induced precipitation of a red solid, which was collected by vacuum filtration. The material was purified by preparative HPLC on a C₁₈ Dynamax column (21.4 mm X 25 cm, 60 Å) using a gradient of 10% to 26% acetonitrile in H₂O with 0. 1% TFA. Fractions containing pure product were combined and lyophilized to give the product (40 mg) as a red solid. HPLC: 2.69 min; HRMS theor. [M+H]:695.2313 amu; obs. [M+H] 695.2307 amu; deviation of -0.9 ppm.

Table 3 shows compounds of the invention in accord with structural diagram IX, made by the method of Example 58, utilizing the appropriate peptide fragments.

**Table 3:**

| **Example No.** | **amino acid sequence** |
|---|---|
| **58** | N-Ac-Glu-Gly Gln |
| **59** | N-Ac-Gln |
| **60** | N-Ac-Ala-Thr-Glu-Gly-Gln |
| **61** | N-Ac-Thr-Ala-Thr-Glu-Gly-Gln |
| **62** | N-Ac-Phe-Thr-Ala-Thr-Glu-Gly-Gln |

Example 59: Red solid; HPLC: 2.85 min; HRMS theor. [M+H]: 509.1672 amu; obs. [M+H] 509.1657 amu; deviation of -3 ppm.

Example 60: Red solid; HPLC: 2.69 min ; HRMS theor. [M+H]: 867.3161 amu; obs. [M+H] 867.3145 amu; deviation of -1.8 ppm.

Example 61: Red solid; HPLC: 2.69 min; HRMS theor. [M+H]: 968.3638 amu; obs. [M+H] 968.3619; deviation of -1.9 ppm.

Example 62: Red solid; HPLC: 3.81 min; HRMS theor. [M+H]: 1115.4322 amu; obs. [M+H] 1115.4286 amu; deviation of -3.2 ppm.

Example 63: *N*-(9,10-Dioxo-9,10-dihydro-phenanthren-4-yl)-succinamic acid methyl ester:

Using a BOHDAN Automated RAM Synthesizer, to a solution 4-amino-phenanthrene-9,10-dione (50 mg, 238 µmol) in THF (10 mL) an excess of Na₂CO₃ (1g) was added, followed by 3-carbomethoxypropionyl chloride (381 µmol, 323 µL). The reaction cycle, which consisted of vortexing the mixture twice for 10 s, was repeated 10 times, and this was followed by filtering and evaporating the solvent. The reaction mixture was then purified on a 10 g silica gel column, using CH₂Cl₂-5% EtOAC/CH₂Cl₂-10% EtOAC/CH₂Cl₂-20% EtOAC/CH₂Cl₂ as the eluant, to yield the pure *N*-(9,10-Dioxo-9,10-dihydro-phenanthren-4-yl)-succinamic acid methyl ester as a brown solid. ¹H NMR (300 MHz. DMSO-d₆) δ 10.26 (1H, s), 8.43 (1H, d, *J*= 7.8 Hz), 8.01 (1H, dd, *J* = 1.4, 7.8 Hz), 7.92 (1H, dd, *J* = 1.4, 7.4 Hz), 7.75 (1H, ddd, *J =* 1.5, 7.8, 7.8 Hz), 7.68 (1H, dd, *J =* 1.5, 7.8 Hz), 7.53 (1H, d, *J* = 7.8 Hz), 7.50 (1H, d, *J*= 7.5 Hz), 3.62 (3H, d), 2.67 (4H, m); HPLC: 3.57 min.

The compounds of examples 67 to 79 inclusive were prepared by the method of Example 63, utilizing the appropriate acid chloride.

### Example 67: 4-(9,10-Dioxo-9,10-dihydro-phenanthren-4-ylcarbamoyl)-butyric acid methyl ester:

Orange solid; ¹H NMR (300 MHz, DMSO-d₆) δ 10.18 (1H, s), 8.35 (1H, d, *J* = 9 Hz), 7.99 (1H, d, *J* = 6 Hz), 7.93 (1H, d, *J* = 9 Hz), 7.75-7.67 (2H, s), 7.53 (1H, d, *J* = 6 Hz), 7.50 (1H, d, *J* = 9 Hz), 3.62 (3H, s), 2.44-2.37 (4H, m), 1.91-1.82 (2H, m); HPLC: 3.89 min.

### Example 68: 7-(9,10-Dioxo-9,10-dihydro-phenanthren-4-ylcarbamoyl)-heptanoic acid methyl ester:

Orange solid; ¹H NMR (300 MHz, DMSO-d₆) δ 10.14 (1H, s), 8.35 (1H, d, *J*= 8.0 Hz), 7.99 (1H, d, *J* = 7.6 Hz), 7.92 (1H, d, *J* = 7.7 Hz), 7.75-7.66 (2H, m), 7.51 (2H, m), 3.59 (3H, s), 2.37 (2H, t, *J =* 7.2 Hz), 2.31 (2H, t, *J* = 7.6 Hz), 1.58 (4H, m), 1.33 (4H, m); HPLC: 5.81 min.

### Example 69: 9-(9,10-Dioxo-9,10-dihydro-phenanthren-4-ylcarbamoyl)-nonanoic acid methyl ester:

Brown solid; ¹H NMR (300 MHz, DMSO-d₆) δ 10.12 (1H, s), 8.35 (1H, d, *J* = 8.5 Hz), 7.99 (1H, d, *J* = 7.7 Hz), 7.92 (1H, d, *J* = 7.6 Hz), 7.70 (2H, m), 7.52 (1H, d, *J* = 7.6 Hz), 7.50 (1 H, d, *J*= 7.7 Hz), 3.58 (3H, s), 2.37 (2H, t, *J =* 7.0 Hz), 2.29 (2H, t, *J* = 7.5 Hz), 1.61 (2H, m), 1.52 (2H, m), 1.29 (8H, m); HPLC: 7.31 min.

### Example 70: 4-(9,10-Dioxo-9,10-dihydro-phenanthren-2-ylcarbamoyl)-butyric acid methyl ester:

Brown solid; ¹H NMR (300 MHz. DMSO-d₆) δ 10.27 (1H, s), 8.28 (1H, d, *J* = 2.5 Hz), 8.23 (1H, d, *J* = 8.9 Hz), 8.19 (1H, d. *J* = 8.1 Hz), 7.99 (1H, d, *J* = 7.3 Hz), 7.94 (1H, d, *J =* 2.0 Hz), 7.75 (1 H, *dd. J* = 7.7, 7.7 Hz), 7.47 (1 H, dd, *J* = 7.5, 7.5 Hz), 3.60 (3H. s), 2.40 (4H, m), 1.87 (2H, tt, *J=* 7.4, 7.4 Hz); HPLC: 5.58 min.

### Example 71: 7-(9,10-Dioxo-9,10-dihydro-phenanthren-2-ylcarbamoyl)-heptanoic acid methyl ester:

Brown solid; ¹H NMR (300 MHz, DMSO-d₆) δ 10.22 (1H, s), 8.28 (1H, d, *J* = 2.4 Hz), 8.20 (2H, m), 7.98 (2H. m), 7.75 (1H, dd, *J* = 7.3, 7.3 Hz), 7.47 (1 H, *dd, J =* 7.7, 7.7 Hz), 3.30 (3H, s), 2.37-2.28 (4H, m), 1.63-1.52 (4H, m), 1.31 (4H, m); HPLC: 7.26 min.

### Example 72: 9-(9,10-Dioxo-9,10-dihydro-phenanthren-2-ylcarbamoyl)-nonanoic acid methyl ester:

Brown solid; ¹H NMR (300 MHz. DMSO-d₆) δ 10.22 (1H, s), 8.28 (1H, d, *J* = 2.0 Hz), 8.22 (1H, d, *J* = 8.9 Hz), 8.19 (1H, d, *J* = 8.2 Hz), 7.98 (2H, m), 7.75 (1H, *dd, J =* 7.3, 7.3 Hz), 7.47 (1H, dd, *J* = 7.7, 7.7 Hz), 3.57 (3H, s), 2.34 (2H, t, *J* = 7.2 Hz), 2.28 (2H, t, *J* = 7.3 Hz), 1.61 (2H, m), 1.52 (2H, m); HPLC: 8.57 min.

### Example 73: N-[5-(3-Methoxycarbonyl-propionylamino)-9,10-dioxo-9,10-dihydrophenanthren-2-yl]-succinamic acid methyl ester:

Pink solid; ¹H NMR (300 MHz, DMSO-d₆) δ 10.40 (1H, s), 10.18 (1H, s), 8.39 (1H, d, *J* = 8.7 Hz). 8.23 (1H, d, *J* = 2.0 Hz), 7.90 (2H, m). 7.63 (1H, d, *J* = 7.9 Hz), 7.45 (1 H, dd, *J* = 7.7, 7.7 Hz), 3.62 (3H, s), 3.61 (3H, s), 2.65 (8H, m); HPLC: 3.64 min.

### Example 74: 4-[5-(4-Methoxycarbonyl-butyrylamino)-9,10-dioxo-9,10-dihydrophenanthren-2-ylcarbamoyl]-butyric acid methyl ester:

Brown solid; ¹H NMR (300 MHz, DMSO-d₆) δ 10.31 (1H, s), 10.11 (1H, s), 8.29 (1H, d, *J* = 8.8 Hz), 7.91 (2H, m), 7.64 (1H, d, *J* = 7.2 Hz), 7.47 (1H, dd, *J* = 7.7, 7.7 Hz), 3.63 (3H, s), 3.60 (3H, s), 2.40 (8H, m), 1.87 (4H, m): HPLC: 4.24 min.

### Example 75: 7-[5-(7-Methoxycarbonyl-heptanoylamino)-9,10-dioxo-9,10-dihydrophenanthren-2-ylcarbamoyl]-heptanoic acid methyl ester:

Brown solid; ¹H NMR (300 MHz, DMSO-d₆) δ 10.23 (1H, s), 10.03 (1H, s), 8.29 (1H, d, *J* = 8.9 Hz), 8.20 (1H, s), 7.90 (2H, m), 7.63 (1H, d, *J* = 8.1 Hz), 7.44 (1H, dd, *J* = 7.7, 7.7 Hz), 3.58 (6H, s), 2.31 (8H, m), 1.56 (8H, m), 1.30 (8H, m); HPLC: 7.09 min.

### Example 76: 9-[5-(9-Methoxycarbonyl-nonanoylamino)-9,10-dioxo-9,10-dihydrophenanthren-2-ylcarbamoyl]-nonanoic acid methyl ester:

Brown solid; ¹H NMR (300 MHz, DMSO-d₆) δ 10.22 (1H, s), 10.03 (1H, s), 8.29 (1H, d, *J =* 8.6 Hz), 8.20 (1H, s), 7.93 (1H, dd, *J =* 2.0. 8.9 Hz), 7.89 (1H, d, *J* = 7.6 Hz). 7.62 (1H, d, *J* = 7.6 Hz), 7.44 (1H, dd,*J*= 7.7, 7.7 Hz), 3.57 (6H, s), 2.38-2.26 (8H, m). 1.62-1.50 (8H, m), 1.28 (16H, m); HPLC: 9.21 min.

### Example 77: 4-(9,10-Dioxo-9,10-dihydro-phenanthren-3-ylcarbamoyl)-butyric acid methyl ester:

Orange solid; ¹H NMR (300 MHz, DMSO-d₆) δ 10.46 (1H, s), 8.53 (1H, d, *J* = 1.7 Hz), 8.04-7.98 (3H, m), 7.83 (1H, dd, *J* = 7.3, 7.3 Hz), 7.67 (1H. dd, *J* = 1.5, 8.8 Hz), 7.56 (1H, dd, *J* = 7.2, 7.2 Hz), 3.61 (3H, s), 2.45 (4H, m). 1.89 (2H, tt, *J =* 7.7, 7.7 Hz); HPLC: 5.68 min.

### Example 78: 8-(9,10-Dioxo-9,10-dihydro-phenanthren-3-ylcarbamoyl)-heptanoic acid methyl ester:

Orange solid; ¹H NMR (300 MHz, DMSO-d₆) δ 10.42 (1H, s), 8.54 (1H, d, *J* = 1.6 Hz), 8.04-7.98 (3H, m), 7.83 (1H. ddd, *J* = 1.2, 7.6. 7.6 Hz), 7.68 (1H, dd, *J* = 1.4, 8.6 Hz). 7.56 (1H, dd, *J* = 7.4. 7.4 Hz), 3.58 (3H, s), 2.40 (2H. t, *J* = 7.3 Hz), 2.30 (2H, t, *J* = 7.3 Hz), 1.63 (2H, m), 1.54 (2H, m), 1.32 (4H, m); HPLC: 7.44 min.

### Example 79: 9-(9,10-Dioxo-9,10-dihydro-phenanthren-3-ylcarbamoyl)-nonanoic acid methyl ester:

Brown solid; ¹H NMR (300 MHz, DMSO-d₆) δ 10.42 (1H, s), 8.54 (1H, s), 8.02 (3H, m), 7.84 (1H, dd, *J* = 7.7, 7.7 Hz). 7.69 (1H, d, *J* = 8.7 Hz), 7.56 (1H, dd, *J* = 7.7, 7.7 Hz), 3.57 (3H, s), 2.40 (2H, t, *J* = 7.3 Hz), 2.28 (2H, t, *J* = 7.3 Hz), 1.63 (2H, m), 1.52 (2H, m), 1.29 (8H, m); HPLC: 8.82 min.

Compounds of examples 80 and 81 were made as follows: To a solution of 2-amino-phenanthrene-9,10-dione (670 mg, 3.0 mmol) in THF (70 mL) diisopropylethylamine (1.1 mL, 6.0 mmol) was added, followed by methyl 4-chloro-4-oxobutyrate for Example 81 *N*-(9,10-dioxo-9,10-dihydro-phenanthren-2-yl)-succinamic acid methyl ester (490 µL, 4.0 mmol) or acetyl chloride for Example 82, *N*-(9,10-dioxo-9,10-dihydro-phenanthren-2-yl)-acetamide (284 µL, 4.0 mmol). Each reaction was stirred for 2.5 h, then the THF was removed by rotary evaporation, and the reaction mixture was chromarographed on silica gel (9:1, CH₂Cl₂: MeOH, v/v) and the product eluted.

### Example 80: N-(9,10-Dioxo-9,10-dihydro-phenanthren-2-yl)-succinamic acid methyl ester:

Brown solid; ¹H NMR (300 MHz, DMSO-d₆) δ 10.39 (1H, s), 8.29 (1H, d, *J=* 2.3 Hz), 8.23 (1H, d, *J=* 9.1 Hz), 8.19 (1H, d, *J =* 8.0 Hz), 7.99 (1H, dd, *J* = 1.4,7.8 Hz), 7.92 (1H, dd, *J=* 2.3, 8.7 Hz), 7.74 (1H, ddd, *J*= 1.1, 7.7, 7.7 Hz), 7.47 (1H, dd, *J* = 7.1, 7.1 Hz), 3.61 (3H, s), 2.65 (4H, m); HPLC: 4.61 min.

### Example 81: N-(9,10-Dioxo-9,10-dihydro-phenanthren-2-yl)-acetamide:

Purple solid; ¹H NMR (300 MHz, DMSO-d₆) δ 10.33 (1H, s), 8.26 (1H, d, *J* = 2.7 Hz), 8.23 (1H, d, *J* = 9.1 Hz), 8.19 (1H, d, *J* = 8.0 Hz), 7.99 (1H, dd, *J* = 1.1, 7.7 Hz), 7.95 (1H, dd, *J* = 2.7, 8.5 Hz), 7.75 (1H, ddd, *J* = 1.6, 8.0, 8.0 Hz), 7.47 (1H, dd, *J* = 7.2, 7.2 Hz), 2.10 (3H, s); HPLC: 4.42 min.

### Example 82: N-[7-(3-Methoxycarbonyl-propionylamino)-9,10-dioxo-9,10-dihydrophenanthren-2-yl]-suceinamic acid methyl ester:

The compound of example 82 was synthesized by a method that differed from that of example 80 only in that double the amount of acid chloride, methyl 4-chloro-4-oxobutyrate, was used and 2,7-diamino-phenauthrene-9,10-dione was used in place of 2-amino-phenanthrene-9,10-dione. Purple solid; ¹H NMR (300 MHz, DMSO-d₆) δ 10.34 (2H, s), 8.25 (2H, d, *J=* 1.9 Hz), 8.10 (2H, d, *J* = 9.0 Hz), 7.89 (2H, dd, *J=* 1.7, 8.4 Hz), 3.61 (6H, s), 2.50 (8H, m); HPLC: 4.78 min.

### Example 90: N-(9,10-Dioxo-9,10-dihydro-phenanthren-3-yl)-succinamic acid methyl ester:

To a solution of 3-amino-phenanthrene-9,10-dione (400 mg, 1.8 mmol) in dioxane (100 mL) was added a large excess of Na₂CO₃ followed by methyl 4-chloro-4-oxobutyrate (240 µL, 2.0 mmol). The reaction was stirred for several hours, then filtered and the solvent was removed under reduced pressure. The material was chromatographed on silica gel with 20% EtOAc in CH₂Cl₂ as eluant and dried to give *N*-(9,10-Dioxo-9,10-dihydro-phenanthren-3-yl)-succinamic acid methyl ester as an orange solid. ¹H NMR (300 MHz, DMSO-d₆) δ 10.60 (1H, s), 8.55 (1H, d, *J*= 1.5 Hz), 8.04-7.98 (3H, m), 7.84 (1H, ddd, *J*= 1.6, 7.9, 7.9 Hz), 7.65 (1H, dd, *J* = 2.0, 8.7 Hz), 7.56 (1H, dd, *J* = 7.6, 7.6 Hz), 3.62 (3H, s), 2.72 (2H, m), 2.67 (2H, m); HPLC: 5.30 min.

### Example 91: N-(9,10-Dioxo-9,10-dihydro-phenanthren-1-yl)-succinamic acid methyl ester:

To a solution of 1-amino-phenanthrene-9,10-dione (50 mg, 240 µmol) in THF (50 mL) was added Na₂CO₃ (210 mg, 2.2 mmol), followed by methyl 4-chloro-4-oxobutyrate (60 µL, 500 µmol). The mixture was stirred overnight, filtered, and diluted with ethyl acetate. The organic layer was washed with water, brine, dried over Mg₂SO₄, filtered, and chromatographed on silica gel with ethyl acetate as the eluant to yield *N*-(9,10-dioxo-9,10-dihydro-phenanthren-1-yl)-succinamic acid methyl ester as an orange solid. ¹H NMR (300 MH₂, DMSO-d₆) δ 12.04 (1H, s), 8.60 (1H, d, *J* = 7.7 Hz), 8.32 (1H, d, *J =* 7.7 Hz), 8.03 (2H, m), 7.79 (2H, m), 7.56 (1H, dd, *J* = 7.2, 7.2 Hz), 3.61 (1H, dd, *J*= 7.2 Hz), 2.81 (2H, m), 2.69 (2H, m); HPLC: 6.49 min. 2.8 Hz), 6.91 (1H, dd, *J* = 2.5, 8.5 Hz), 5.87 (2H, s). Anal. Calcd for C₁₄H₉NO₂-0.33 H₂O C, 73.35 4.25 N, 6.11. Found: C, 73.33, 73.39; H, 4.22, 4.21; N, 6.28, 6.29.

### Example 88: 3-Amino-phenanthrene-9,10-dione:

Brown solid; ¹H NMR (300 MHz, DMSO-d₆) δ 7.99 (2H, m), 7.83 (1H, d, *J* = 8.4 Hz), 7.78 (1H, d, *J* = 7.2 Hz), 7.53 (1 H, dd, *J* = 7.2, 7.2 Hz), 7.30 (1H, s), 6.89 (2H, s), 6.64 (1H, dd, *J* = 1.5, 8.6 Hz). Anal. Calcd. For C₁₄H₉O₂N-0.10 H₂O C, 74.72; H, 4.12; N, 6.22 Found: C, 74.94, 74.74; H, 4.10, 4.08; N, 6.26, 6.30.

### Example 89: 4-Amino-phenanthrene-9,10-dione:

Black solid; ¹H NMR (300 MHz. DMSO-d₆) δ 8.56 (1H, d, J = 8.0 Hz), 7.94 (1H, dd, J = 1.4, 7.8 Hz), 7.73 (1H, ddd, J = 1.6, 7.8, 7.8 Hz), 7.41 (1H, ddd, J = 1.0, 7.9, 7.9 Hz), 7.36 (1H, dd, J = 3.2, 5.9 Hz), 7.20 (2H, m), 5.84 (2H, s). Anal. Calcd. For C₁₄H₉NO₂ C, 75.33; H, 4.06; N, 6.27 Found: C, 75.12, 75.12; H, 4.28, 4.29; N, 6.21, 6.20.

### Example 90: N-(9,10-Dioxo-9,10-dihydro-phenanthren-3-yl)-succinamic acid methyl ester:

To a solution of 3-amino-phenanthrene-9,10-dione (400 mg, 1.8 mmol) in dioxane (100 mL) was added a large excess of Na₂CO₃ followed by methyl 4-chloro-4-oxobutyrate (240 µL, 2.0 mmol). The reaction was stirred for several hours, then filtered and the solvent was removed under reduced pressure. The material was chromatographed on silica gel with 20% EtOAc in CH₂Cl₂ as eluant and dried to give *N*-(9,10-Dioxo-9,10-dihydro-phenanthren-3-yl)-succinamic acid methyl ester as an orange solid. ¹H NMR (300 MHz. DMSO-d₆) δ 10.60 (1H, s), 8.55 (1H, d, *J* = 1.5 Hz), 8.04-7.98 (3H, m), 7.84 (1H, ddd, *J* = 1.6, 7.9, 7.9 Hz), 7.65 (1H, dd, *J* = 2.0, 8.7 Hz), 7.56 (1H, dd, *J* = 7.6, 7.6 Hz), 3.62 (3H, s), 2.72 (2H, m), 2.67 (2H, m); HPLC: 5.30 min.

### Example 91: N-(9,10-Dioxo-9,10-dihydro-phenanthren-1-yl)-succinamic acid methyl ester:

To a solution of 1-amino-phenanthrene-9,10-dione (50 mg, 240 µmol) in THF (50 mL) was added Na₂CO₃ (210 mg. 2.2 mmol), followed by methyl 4-chloro-4-oxobutyrate (60 µL, 500 µmol). The mixture was stirred overnight, filtered, and diluted with ethyl acetate. The organic layer was washed with water, brine, dried over Mg₂SO₄, filtered, and chromatographed on silica gel with ethyl acetate as the eluant to yield *N*-(9,10-dioxo-9,10-dihydro-phenanthren-1-yl)-succinamic acid methyl ester as an orange solid. ¹H NMR (300 MHz, DMSO-d₆) δ 12.04 (1H, s), 8.60 (1H, d, *J* = 7.7 Hz), 8.32 (1H, d, *J* = 7.7 Hz), 8.03 (2H, m), 7.79 (2H, m), 7.56 (1H, dd, *J* = 7.2, 7.2 Hz), 3.61 (1H, dd, *J* = 7.2 Hz), 2.81 (2H, m), 2.69 (2H, m); HPLC: 6.49 min.

### Examples 92 to 95:

Compounds of examples 92 to 95 inclusive were prepared substantially in accordance with the procedures disclosed in Schmidt, J. *Chem. Ber*. **1903,** *23*, 3726-3730, taking into account the results disclosed by Ray, F. E.; Francis, W. C. *J. Org. Chem*. **1943,** *8*, 52-59, which procedures and results are incorporated herein by reference.

### Example 92: 2-Nitro-phenanthrene-9,10-dione:

Orange solid; ¹H NMR (300 MHz, DMSO-d₆) δ 8.80 (1H, d. *J* = 2.8 Hz). 8.69 (1H, d, *J* = 8.5 Hz), 8.60 (1H, dd, *J* = 2.4. 8.7 Hz), 8.51 (1H, d, *J* = 8.3 Hz), 8.22 (1H, dd, *J* = 1.4, 7.7 Hz), 7.93 (1H, ddd, *J* = 1.6, 8.1. 8.1 Hz), 7.73 (1H, dd, *J* = 7.5, 7.5 Hz). Anal. Calcd. For C₁₄H₇NO₄: C, 66.41; H. 2.79; N. 5.53; Found: C. 66.70. 66.71; H, 2.83, 2.84; N, 5.57, 5.63.

### Example 93: 4-Nitro-phenanthrene-9,10-dione:

Orange solid; ¹H NMR (300 MHz, DMSO-d₆) δ 8.27 (1H, dd, *J* = 1.5, 7.9 Hz), 8.17 (1H, dd, *J* = 1.3, 7.9 Hz), 8.10 (1H, dd, *J* = 1.5, 7.8 Hz), 7.79-7.71 (2H, m), 7.63 (1H, ddd, *J* = 0.9, 7.7, 7.7 Hz), 7.46 (1 H, d, *J* = 8.0 Hz). Anal. Calcd. For C₁₄H₇NO₄-0.25 H₂O C, 65.26; H, 2.93; N, 5.43. Found: C, 65.19, 65.24; H. 2.82, 2.83; N, 5.43, 5.44.

### Example 94: 2,7-Dinitro-phenanthrene-9,10-dione:

Yellow solid; ¹H NMR (300 MHz, tfashake-DMSO-d₆) δ 8.77 (2H. d, *J* = 2.7 Hz), 8.76 (2H, d, *J* = 9.1 Hz), 8.62 (2H, dd, *J* = 2.7, 9.1 Hz). Anal. Calcd. for C₁₄H₆N₂O₆ C, 56.39; H, 2.02; N, 9.39 Found: C. 56.20, 56.44; H, 2.17, 2.14; N, 8.89, 8.95.

### Example 95: 2,5-Dinitro-phenanthrene-9,10-dione:

Yellow solid; ¹H NMR (300 MHz, DMSO-d₆) δ 8.67 (1H, d, J = 2.5 Hz). 8.54 (1H, dd, J = 2.5, 9.1 Hz), 8.36 (1H, dd, J = 1.5, 8.1 Hz), 8.26 (1H, dd, J = 1.3, 7.8 Hz), 7.87 (1H, dd, J = 7.8, 7.8 Hz), 7.73 (1H, d. J = 8.8 Hz).

### Example 96: 2-Bromo-phenanthrene-9,10-dione:

This compound was prepared substantially in accordance with the procedures disclosed in Bhatt, M. V.; *Tetrahedron* **1964,** *20*, 803-821, which procedures are incorporated herein by reference. Orange solid; ¹H NMR (300 MHz, DMSO-d₆) δ 8.30 (1H, d, *J* = 7.9 Hz), 8.27 (1H, d, *J* = 8.6 Hz), 8.07 (1H, d, *J* = 2.4 Hz), 8.04 (1H, d, *J* = 1.0, 7.6 Hz), 7.96 (1H, dd, *J* = 2.7, 8.9 Hz), 7.79 (1H, ddd, *J =* 1.7, 8.2, 8.2 Hz). 7.56 (1H, dd, *J* = 7.6, 7.6 Hz). Anal. Calcd. For C₁₄H₇O₂ C, 58.57; H. 2.46. Found: C, 58.22, 58.29; H, 2.63, 2.65.

### Examples 97 and 98:

The compounds of examples 97 and 98 were prepared substantially in accordance with the procedures disclosed in Langenbeck, W.; Schaller, R.; Arneberg, K. *Chem. Ber*. 1942, *75*, 1483-1488, which procedures are incorporated herein by reference.

### Example 97: 9,10-Dioxo-9,10-dihydro-phenanthrene-2-carboxylic acid:

Yellow solid; ¹H NMR (300 MHz, DMSO-d₆) δ 13.45 (1H, s), 8.51 (1H, d, *J* = 1.9 Hz), 8.46 (1H, d, *J*= 8.2 Hz), 8.37 (1H, d, *J* = 8.0 Hz), 8.24 (1H, dd, *J*= 2.0, 8.2 Hz), 8.08 (1H, dd, *J=* 0.75, 7.6 Hz), 7.83 (1H, dd, *J* = 7.2, 7.2 Hz), 7.61 (1H, dd, *J* = 7.5, 7.5 Hz) Anal calcd. For C₁₅H₈O₄-0.4 H₂O C, 69.45; H, 3.42 Found: C, 69.59, 69.66; H 3.16, 3.17.

### Example 98: 9,10-Dioxo-9,10-dihydro-phenanthrene-3-carboxylic acid:

Orange solid; ¹H NMR (300 MHz, DMSO-d₆) δ 13.66 (1H, br s), 8.71 (1H, s), 8.35 (1H, d, *J* = 8.0 Hz), 8.12 (1H, d, *J* = 8.0 Hz), 8.08-8.02 (2H, m), 7.81 (1H, dd, *J* = 7.5, 7.5 Hz), 7.58 (1H, dd, *J* = 7.5, 7.5 Hz). Anal. Calcd. For C₁₅H₈O₄-0.2 H₂O C, 70.42; H, 3.31 Found: C, 70.61, 70.76; H, 3.41, 3.44.

### Examples 100 and 101:

The compounds of examples 100 and 101 were prepared substantially in accordance with the procedures disclosed in Mosettig, E.; Van de Kamp, J. *J. Am. Chem. Soc.* **1930**, *52*, 3704-3710, which procedures are incorporated herein by reference.

### Example 100: 3-Acetyl-phenanthrene-9,10-dione:

Orange solid; ¹H NMR (300 MHz, DMSO-d₆) δ 8.70 (1H, d, *J=* 2.4 Hz), 8.45 (1H, d, *J* = 8.1 Hz), 8.13 (1H, d, *J =* 8.1 hz), 8.07 (1H, dd, *J* = 2.4, 7.8 Hz), 8.02 (1H, dd, *J*= 1.5, 8.1 Hz), 7.82 (1H, ddd, *J*= 2.4, 7.6, 7.6 Hz), 7.58 (1H, ddd, *J*= 1.2, 7.5, 7.5 Hz), 2.75 (3H, s); HPLC: 5.48 min.

### Example 101: 2-Acetyl-phenanthrene-9,10-dione:

Yellow solid; ¹H NMR (300 MHz, DMSO-d₆) δ 8.49-8.45 (2H, m), 8.39 (1H, d, *J* = 8.1 Hz), 8.27 (1H, dd, *J* = 2.1, 8.4 Hz), 8.08 (1H, dd, *J =* 1.2, 7.8 Hz), 7.83 (1H, ddd, *J =* 1.5, 7.4, 7.4 Hz), 7.61 (1H, dd, *J* = 7.5 Hz), 2.68 (3H, s); HPLC: 5.27 min.

### Example 102:

To a solution of 9,10-dioxo-9,10-dihydro-phenanthrene-3-carboxylic acid (1.1 g, 4.5 mmol) in anhydrous DMF (10 mL) under N₂ was added t-butyl glycine hydrochloride (760 mg, 4.6 mmol), EDC (1.1 g, 6.0 mmol), DMAP (60 mg, 500 µmol) and diisopropylethylamine (2.1 mL, 12 mmol) and the resultant solution was stirred for 16 h. The reaction was diluted with EtOAc and washed with 1 M HCl, water, sat'd aq. NaHCO₃ and brine. The organic layer was dried over MgSO₄, filtered, concentrated, and chromatographed on silica gel (3:1 EtOAc:CH₂Cl₂, v/v) to afford a yellow solid (500 mg). This solid was subsequently recrystallized from refluxing EtOAc to afford pure [(9,10-dioxo-9,10-dihydro-phenanthrene-3-carbonyl)-amino]-acetic acid tert-butyl ester (310 mg, 31 %). Yellow solid; ¹H NMR (300 MHz, DMSO-d₆) δ 9.29 (1H, dd, *J* = 6.1, 6.1 Hz), 8.71 (1H, s), 8.38 (1H, d, *J* = 8.0 Hz), 8.13 (1H, d, *J* = 8.3 Hz), 8.07 (1H, dd, *J* = 1.5, 7.9 Hz), 7.97 (1H, dd, *J* = 1.5, 8.3 Hz), 7.85 (1 H, *ddd, J*= 1.5, 7.8, 7.8 Hz), 7.58 (1H, ddd, *J*= 7.2, 7.2 Hz), 4.00 (2H, d, *J* = 6.1 Hz), 1.45 (9H, s); Anal. Calcd. For C₂₁H₁₉NO₅-0.2H₂O: C, 68.36; H, 5.30; N, 3.80. Found: C, 68.22, 68.58; H, 5.17, 5.20; N, 3.87, 3.89.

The compound of example 103 was prepared by the method of Example 102, utilizing 9,10-dioxo-9,10-dihydro-phenanthrene-2-carboxylic acid as the starting material.

### Example 103: [(9,10-Dioxo-9,10-dihydro-phenanthrene-2-carbonyl)-amino]-acetic acid tert-butyl ester:

Yellow solid; ¹H NMR (300 MHz, DMSO-d₆) δ 9.23 (1H, dd, *J* = 5.7, 5.7 Hz). 8.54 (1H, d, *J* = 1.9 Hz), 8.46 (1H, d, *J* = 8.7 Hz), 8.39 (1H, d, *J =* 7.9 Hz), 8.22 (1H, dd, *J* = 2.3, 8.7 Hz), 8.07 (1H, dd, *J =* 1.6, 7.7 Hz), 7.82 (1H, ddd, *J =* 1.5, 7.9, 7.9 Hz). 7.59 (1H, dd, *J* = 7.4, 7.4 Hz), 3.94 (2H. d, *J* = 6.3 Hz). 1.44 (9H, s); Anal. Calcd. For C₂₁H₁₉NO₅-0.1H₂O: C, 68.69; H, 5.27; N, 3.81. Found: C. 68.67, 68.84; H, 5.38, 5.41; N, 3.77. 3.78.

### Example 104:

To a solution of [(9,10-dioxo-9,10-dihydro-phenanthrene-3-carbonyl)-amino]-acetic acid tert-butyl ester (200 mg, 550 µmol) in CH₂Cl₂ (10 mL) under N₂ was added TFA (10 mL). This mixture was stirred for 2 h and concentrated under reduced pressure. The residue was dissolved in CH₂Cl₂ and evaporated once again to rid residual TFA. The material was recrystallized from refluxing EtOAc to afford [(9,10-dioxo-9,10-dihydro-phenanthrene-3-carbonyl)-amino]-acetic acid (70 mg, 47 %). Orange solid; ¹H NMR (300 MHz, DMSO-d₆) δ 12.7 (1H, s), 9.29 (1H, dd, *J* = 5.7, 5.7 Hz), 8.71 (1H, s), 8.39 (1 H, d, *J* = 7.9 Hz), 8.13 (1H, d, *J* = 8.3 Hz), 8.07 (1H, dd, *J* = 1.2, 7.5 Hz), 7.98 (1H, dd, *J* = 1.6, 8.2 Hz), 7.85 (1 H, ddd, *J* = 1.3, 8.7, 8.7 Hz), 7.59 (1 H, dd, *J* = 7.5, 7.5 Hz), 4.03 (2H, d, *J* = 5.6 Hz); Anal. Calcd. For C₁₇H₁₁NO₅-1.0H₂O: C, 62.39; H, 4.00; N, 4.28. Found: C, 62.48, 62.36; H, 3.63, 3.61; N, 4.46, 4.47.

The compound of example 105 was prepared by the method of example 104, by utilizing [(9,10-dioxo-9,10-dihydro-phenanthrene-2-carbonyl)-amino]-acetic acid tert-butyl ester as the starting material.

### Example 105: [(9,10-Dioxo-9,10-dihydro-phenanthrene-2-carbonyl)-amino]-acetic acid:

Orange solid; ¹H NMR (300 MHz, DMSO-d₆) δ 12.69 (1H, s), 9.22 (1H, dd, *J* = 6.0, 6.0 Hz), 8.54 (1H, d, *J* = 1.9 Hz), 8.46 (1 H, d, *J* = 8.2 Hz), 8.39 (1 H, d, *J* = 8.0 Hz), 8.23 (1 H, dd, *J* = 1.9, 8.4 Hz), 8.08 (1H, dd, *J* = 1.1, 7.5 Hz), 7.82 (1H, ddd, *J* = 1.5, 8.0, 8.0 Hz), 7.59 (1H, dd, *J* = 8.0, 8.0 Hz), 3.97 (2H, d, *J* = 5.8 Hz); Anal. Calcd. For C₁₇H₁₁NO₅: C, 66.02; H, 3.58; N, 4.53. Found: C, 66.09, 66.28; H, 3.69, 3.71; N, 4.59, 4.59.

### Example 106: 9,10-Phenanthrenedione was purchased from Aldrich Chemical Company, Milwaukee, WI and used as received.

### Assays for Biological Activity

### Method A:

### Phosphatase assay using pNPP as substrate:

CD45 enzyme was obtained from BIOMOL (Plymouth Meeting, PA). Phosphatase activity was assayed in a buffer containing final concentrations of 25 mM imidazole at pH 7.0, 50 mM NaCl, 2.5 mM ethylenediaminetetraacetic acid ("EDTA"), 5 mM dithiothreitol ("DTT") and 10 µg/mL bovine serum albumin ("BSA") using *p*NPP as a substrate. Compounds were tested in a range from 30 to 0.01 µM, with a final concentration of I or 5% dimethylsulfoxide ("DMSO"), depending on the compound solubility. Activity was measured by following the increase in absorbance at 405 nm using a SpectraMax Plus spectrophotometric plate reader (Molecular Devices, Sunnyvale, CA).

### Method B:

### Cytotoxicity Assay:

Calcein-AM (Molecular Probes, Eugene, OR) uptake, as a quantitative measure of cell viability, was used to evaluate the toxic effect of compounds on T cells. Briefly, PBMC were treated for 3-7 days with 3-10 µg/ml PHA, a potent T-cell mitogen, to preferentially expand the T-cell population. (Bradley, Linda M. *Cell Poliferation* in *Selected Methods in Cellular Immunology*, Eds. Mishell, B.B. and Shiigi, S.M., W.H. Freeman and Co., San Francisco, 1980.)

The T-cell lymphoblasts were purified by separation over Lymphoprep, plated at 2 x 10⁵/well in a round bottom 96-well plate containing RPMI with compound and incubated overnight at 37 °C in an incubator containing 5% CO₂. The dilution scheme and culture media were the same as those used in the T-cell proliferation assay. After the incubation period, cells were washed with Dulbecco's phosphate- buffered saline (D-PBS) and incubated with 1µM Calcein-AM for 30-45 min in D-PBS as described in the technical sheet provided with The LIVE/DEAD Viability/Cytotoxicity Kit from Molecular Probes. Percent viability was assessed on a fluorescent plate reader (excitation filter 485/20 nm; emission filter 530/25 nm) where the 100% control value is the fluorescence intensity observed in the absence of test compound.

### Method C:

### Phosphatase assay using lck 10-mer as substrate:

Phosphatase activity was assayed in 96 well plates in a buffer containing final concentrations of 25 mM HEPES at pH 7.2, 5 mM DTT and 10 µg/mL BSA. using the *lck* carboxy-terminal peptide TEGQpYQPQP as the substrate (Cho. H., Krishnaraj, R., Itoh, M., Kitas, E., Bannwarth, W., Saito, H.. Walsh, C.T. 1993. Substrate specificities of catalytic fragments of protein tyrosine phosphatases (HPTPb, LAR. and CD45) toward the phosphotyrosylpeptide substrates and thiophosphotyrosylated peptides as inhibitors. *Protein Science* 2:977-984). Compounds were tested in a range from 30 to 0.01 µM in a final concentration of 5% DMSO. Enzyme was incubated with substrate, with or without compound, at room temperature for 1.5 h. At the end of the incubation period, BIOMOL "Green Reagent" (BIOMOL, Plymouth Meeting. PA) was added to each well, the plates incubated at room temperature for 30 min and absorbance read at 620 nm.

### Method D:

### Cell isolmion and T cell proliferation assay:

Whole blood was obtained from healthy human blood donors. Peripheral blood mononuclear cells ("PBMC") were isolated using Lymphoprep density-gradient centrifugation (Nycomed Amersham, Oslo, Norway), washed, counted and resuspended at 2 X 10⁶ cells/mL in RPMI 1640 medium containing glutamine, 0.1 mg/mL gentamycin and 10% heat inactivated human serum. PBMC were transferred to 96-well plates (2 X 10⁵ cells/well) containing compound or vehicle control, with the final concentration of DMSO not to exceed 0.3%, and incubated for 1 hour before addition of the activating anti-CD3 antibody, OKT3 (30 ng/mL). After 24 hours in culture, the cells were pulsed with [³H]thymidine (1 µCi/well) overnight and harvested the next day onto 96-well Packard GF/C filter plates using a Packard Cell Harvester (Packard Instruments, Meriden, CT). The filter plate was dried, the bottom of the plate sealed, 25 µL of Microscint 20 scintillation fluid added to each well, the top of the plate sealed with TopSeal-A, and the plate counted on a Packard TopCount. The data from the TopCount is transferred into Excel 5 (Microsoft, Redmond, WA) and formatted for EC₅₀ determination using Prism software (GraphPad Software, San Diego, CA).

Table 4 shows the inhibition of CD45 activity in the *p*NPP asssay and the *lck* assay certain compounds of the present invention. Inhibition in the T cell proliferation assay, as well as results from T cell cytotoxicity assay are shown.

**Table 4:**

| **Example. No.** | **pNPP IC**_{**50**} **(µM)** | **lck IC**_{**50**} **(µM)** | **T cell prolif. IC**_{**50**} **(µM)** | **CC**_{**50**} **(µM)** |
|---|---|---|---|---|
| **1** | 0.6 | 11 | 3.1 | 20 |
| **2** | 0.5 | 11 | 1.0 | 14 |
| **3** | 0.7 | 17 | 1.1 | 8.2 |
| **4** | 0.2 | 3.8 | 0.1 | 3.5 |
| **5** | 0.3 | 12 | 1.0 | 8.2 |
| **6** | 0.5 | 16 | 1.5 | 12 |
| **7** | 0.5 | 13 | 0.5 | 9 |
| **8** | 0.4 | 15 | 1.4 | 12 |
| **9** | 0.8 | 5.2 | 0.8 | 9.1 |
| **10** | 1.3 | 4.9 | 0.7 | 10 |
| **11** | 2.8 | 11.6 | 6.5 | >30 |
| **12** | 1.3 | 6.0 | 3.0 | 15 |
| **13** | 1.1 | 5.8 | 1.5 | 14 |
| **14** | 12.9 | >30 | 0.8 | 5.3 |
| **15** | 1.0 | 15 | 6.4 | >30 |
| **16** | 3.7 | >30 | 0.8 | 3.6 |
| **17** | 0.8 | >30 | 1.8 | 7.3 |
| **18** | 0.75 | >30 | 1.3 | 8.4 |
| **19** | 4.49 | >30 | 1.6 | 4.4 |
| **20** | 0.99 | >30 | 2.1 | 8.9 |
| **21** | 0.99 | >30 | 1.8 | 4.9 |
| **22** | 3.6 | 9.7 | 0.7 | >30 |
| **23** | 1.1 | 3.0 | 20 | >30 |
| **24** | 0.6 | 2.4 | >30 | >30 |
| **25** | 1.2 | 2.5 | >30 | >30 |
| **26** | 0.7 | 2.3 | >30 | >30 |
| **27** | 0.7 | 3.1 | >30 | >30 |
| **28** | 1.1 | 2.2 | 5 | >30 |
| **29** | 1.1 | 2.5 | 20 | >30 |
| **30** | 0.9 | 1.7 | 14.5 | >30 |
| **31** | 0.6 | 2.5 | 7 | >30 |
| **32** | 0.7 | 2.3 | 10.5 | >30 |
| **33** | 1.1 | 2.5 | 16 | >30 |
| **34** | 0.9 | 1.9 | >30 | ND |
| **35** | 0.9 | 1.4 | >30 | >30 |
| **36** | 1.0 | 2.2 | >30 | >30 |
| **37** | 1.1 | 2.1 | >30 | >30 |
| **38** | 0.7 | 2.2 | >30 | ND |
| **39** | 0.7 | 2.3 | >30 | >30 |
| **40** | 0.6 | 3.9 | 19.3 | >30 |
| **41** | 1.4 | 3.5 | 8.8 | >30 |
| **42** | 0.9 | 3.5 | 3.3 | >30 |
| **43** | 1.9 | 3.8 | 9.0 | >30 |
| **44** | 1.1 | 3.8 | 14.2 | >30 |
| **45** | 1.4 | 3.9 | 5.0 | >30 |
| **46** | 1.0 | 3.8 | 5.5 | >30 |
| **47** | 0.7 | 4.2 | 3.0 | >30 |
| **48** | 0.6 | 5.7 | 11 | >30 |
| **49** | 0.9 | 6.1 | 16 | >30 |
| **50** | 0.7 | 2.8 | 23 | >30 |
| **51** | 1.1 | 4.8 | 14 | >30 |
| **52** | 0.8 | 3.0 | 20 | >30 |
| **53** | 1.0 | 3.7 | 16 | >30 |
| **54** | 0.9 | 3.9 | 20 | >30 |
| **55** | 1.4 | 4.7 | >30 | >30 |
| **56** | 0.8 | 2.3 | 19 | >30 |
| **57** | 1.3 | 3.2 | 21 | >30 |
| **58** | 1.5 | 17.8 | >30 | ND |
| **59** | 1.1 | 5.8 | >30 | ND |
| **60** | 2.0 | 7.9 | 21.4 | ND |
| **61** | 1.8 | 8.5 | 23 | ND |
| **62** | 1.4 | 15.3 | >30 | ND |
| **63** | 0.7 | 2.6 | 1.2 | 8 |
| **67** | 1.0 | 2.2 | 0.8 | 4 |
| **68** | 1.0 | 3.2 | 1.8 | 7.5 |
| **69** | 0.6 | 4.8 | 4.6 | 14 |
| **70** | 0.4 | 4.7 | 0.5 | 3 |
| **71** | 0.5 | 3.7 | 1.3 | 14 |
| **72** | >30 | ND | ND | ND |
| **73** | 0.5 | 3.0 | 5.0 | 11 |
| **74** | 0.8 | 3.3 | 2.6 | 19 |
| **75** | 0.8 | 10.0 | 2.6 | 13 |
| **76** | 9.3 | >30 | 7.0 | >30 |
| **77** | 0.8 | 2.9 | 1.6 | 9 |
| **78** | 0.7 | 6.9 | 1.3 | 28 |
| **79** | 1.6 | >30 | 0.5 | >30 |
| **80** | 5.9 | 4.9 | 0.7 | 6.5 |
| **81** | 1.2 | 2.5 | 0.2 | 0.9 |
| **82** | 0.5 | 2.9 | 1.1 | 7 |
| **90** | 0.4 | 3.2 | 1.0 | 18 |
| **91** | 0.4 | 4.7 | 0.5 | 9 |
| **97** | 1.0 | 3.3 | >30 | >30 |
| **98** | 1.0 | 2.4 | >30 | ND |
| **100** | 0.8 | 4.2 | 0.8 | 3.5 |
| **101** | 0.8 | 2.9 | 1.1 | 4.5 |
| **102** | 0.6 | 3.4 | 0.3 | 2.5 |
| **103** | 0.6 | 4.9 | 0.4 | 10 |
| **104** | 0.5 | 3.4 | 6.5 | >30 |
| **105** | 0.8 | 2.2 | 4.4 | >30 |
| **106** | 0.7 | 3.1 | 0.3 | 1.3 |

## Claims

1. A compound in accord with structural diagram I, 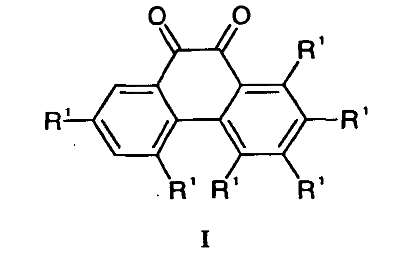 or tautomers thereof or pharmaceutically-acceptable salts thereof,
wherein:
R¹ at each occurrence is independently selected from hydrogen, NH-CO-R², CO-NH-R², Ar, (CH₂)ₙCH(COOH)R³ COR³, NHCOCH₂CH(COOH)NHR⁴ and N(R⁵)₂,
wherein:
R² is selected from (C₁-C₄)alkyl, (C₁-C₈)alkylCOOR⁶ and phenyl, wherein phenyl moieties of R² can be substituted at one, two or three positions with a moiety selected from halogen, NO₂ and CF₃, alkyl moieties of R² can be substituted with halogen, and R⁶ is selected from hydrogen and (C₁-C₄)alkyl;
Ar at each occurrence is independently selected from groups in accord with the following structural diagrams, 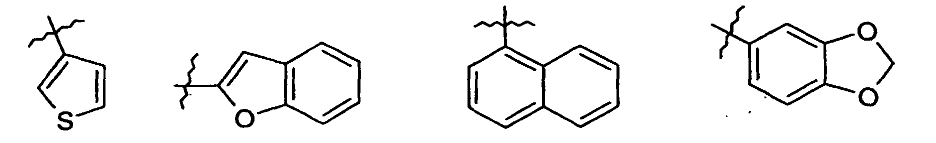 or from phenyl which can be substituted with a moiety selected from halogen, (C₁-C₄)alkyl, O-(C₁-C₄)alkyl and halo(C₁-C₄)alkyl;
R³ at each occurrence is an N-linked oligopeptide selected from GQ-N-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPEG, GRPEG, ERPEG, RRPEG, GGPRG, EGPRG, RGPRG, GEPRG, EEPRG, REPRG, GRPRG, ERPRG and RRPRG,
R⁴ at each occurrence is a C-linked N-acetyl-oligopeptide selected from Q, EGQ, ATEGQ, TATEGQ, and FTATEGQ, and
R⁵ at each occurrence is selected from hydrogen and tosyl;
with the proviso that said compound is not 9,10-dioxo-9,10-dihydrophenanthrene-2-carboxylic acid; 9,10-dioxo-9,10-dihydro-phenanthrene-3-carboxylic acid; 3-acetyl-phenanthrene-9,10-dione; 2-acetyl-phenanthrene-9,10-dione, or 9,10-phenanthrenedione.

2. A compound according to Claim 1, in accord with structural diagram II, 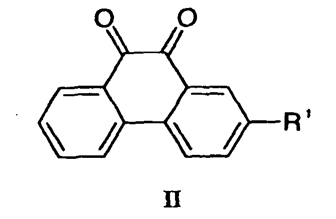 wherein:
R¹ is N(R⁵)₂, where R⁵ is selected from hydrogen and tosyl with the proviso that at least one R⁵ moiety is tosyl.

3. A compound according to Claim 1, in accord with structural diagram III, 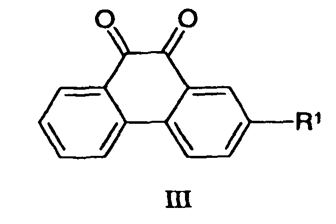 wherein:
R¹ is Ar and Ar is selected from groups in accord with the following structural diagrams: 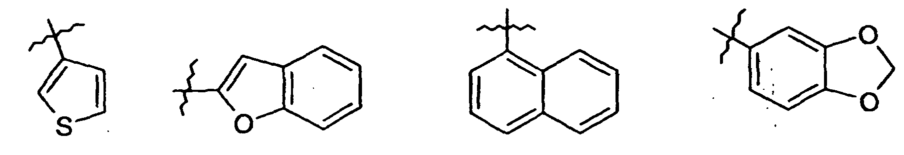 or from phenyl which can be substituted with a moiety selected from halogen, (C₁-C₄)alkyl, O-(C₁-C₄)alkyl and halo(C₁-C₄)alkyl.

4. A compound according to Claim 3, wherein Ar is phenyl substituted with perfluoromethyl.

5. A compound according to Claim 1, in accord with structural diagram IV 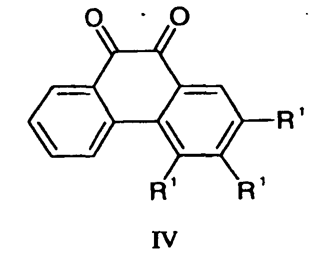 wherein:
R¹ is selected from hydrogen, (CH₂)ₙCH(COOH)R³ and COR³ where R³ is an N-linked peptide selected from GQ-*N*-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPEG, GRPEG, ERPEG, RRPEG, GGPRG, EGPRG, RGPRG, GEPRG, EEPRG, REPRG, GRPRG, ERPRG and RRPRG, with the proviso that no more than one R¹ moiety is other than hydrogen.

6. A compound according to Claim 1, wherein R¹ is selected from hydrogen and NHC(O)(CH₂)ₙCOOCH₃ where n is an integer selected from the range 1 to 8.

7. A pharmaceutical composition comprising an effective amount of a compound according to any one of Claims 1 to 6, and a pharmaceutically-acceptable excipient or diluent.

## Patentansprüche

1. Verbindungen gemäß dem Strukturdiagramm I, 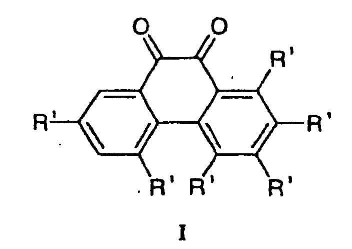 oder Tautomere davon oder pharmazeutisch annehmbare Salze davon,
wobei:
R¹ jeweils unabhängig ausgewählt ist aus Wasserstoff, NH-CO-R², CO-NH-R², Ar, (CH₂)ₙCH(COOH)R³ COR, NHCOCH₂CH(COOH)NHR⁴ und N (R⁵)₂,
wobei:
R² ausgewählt ist aus (C₁-C₄)-Alkyl, (C₁-C₈)-AlkylCOOR⁶ und Phenyl, wobei die Phenylgruppierungen von R² an einer, zwei oder drei Positionen mit einer Gruppierung, ausgewählt aus Halogen, NO₂ und CF₃, substituiert sein können, die Alkylgruppierungen von R² mit Halogen substituiert sein können und R⁶ ausgewählt ist aus Wasserstoff und (C₁-C₄)-Alkyl;
Ar jeweils unabhängig ausgewählt ist aus Gruppen gemäß den folgenden Strukturdiagrammen, 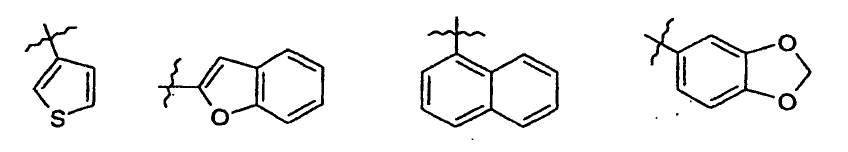 oder aus Phenyl, das mit einer Gruppierung, ausgewählt aus Halogen, (C₁-C₄)-Alkyl, O-(C₁-C₄)-Alkyl und Halogen(C₁-C₄)-Alkyl substituiert sein kann;
R³ jeweils für ein N-verknüpftes Oligopeptid, ausgewählt aus GQ-*N*-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPEG, GRPEG, ERPEG, RRPEG, GGPRG, EGPRG, RGPRG, GEPRG, EEPRG, REPRG, GRPRG, ERPRG und RRPRG, steht,
R⁴ jeweils für ein C-verknüpftes N-Acetyl-Oligopeptid, ausgewählt aus Q, EGQ, ATEGQ, TATEGQ und FTATEGQ, steht und
R⁵ jeweils ausgewählt ist aus Wasserstoff und Tosyl;
mit der Maßgabe, daß es sich bei der Verbindung nicht um 9,10-Dioxo-9,10-dihydro-phenanthren-2-carbonsäure; 9,10-Dioxo-9,10-dihydro-phenanthren-3-carbonsäure, 3-Acetyl-phenanthren-9,10-dion; 2-Acetyl-phenanthren-9,10-dion, oder 9,10-Phenanthrendion handelt.

2. Verbindungen nach Anspruch 1, gemäß dem Strukturdiagramm II, 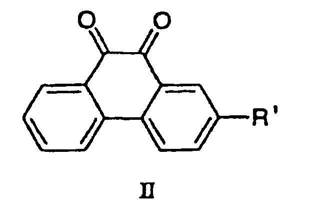 wobei:
R¹ für N(R⁵)₂ steht, worin R⁵ ausgewählt ist aus Wasserstoff und Tosyl, mit der Maßgabe, daß es sich bei wenigstens einer R⁵-Gruppierung um Tosyl handelt.

3. Verbindungen nach Anspruch 1, gemäß dem Strukturdiagramm III, 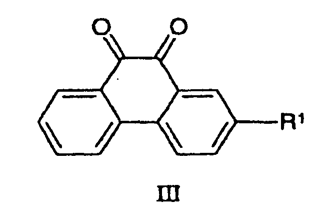 wobei:
R¹ für Ar steht und Ar ausgewählt ist aus Gruppen gemäß den folgenden Strukturdiagrammen: 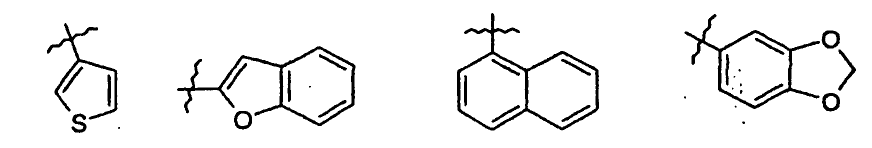 oder aus Phenyl, das mit einer Gruppierung, ausgewählt aus Halogen, (C₁-C₄)-Alkyl, O-(C₁-C₄)-Alkyl und Halogen(C₁-C₄)-Alkyl substituiert sein kann.

4. Verbindungen nach Anspruch 3, wobei es sich bei Ar um mit Perfluormethyl substituiertes Phenyl handelt.

5. Verbindungen nach Anspruch 1, gemäß dem Strukturdiagramm IV, 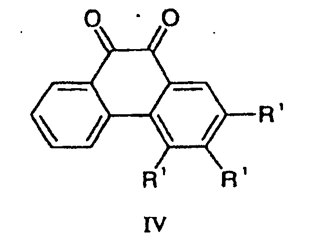 wobei:
R¹ ausgewählt ist aus Wasserstoff, (CH₂)ₙCH(COOH)R³ und COR³, worin R³ für N-verknüpftes Peptid, ausgewählt aus GQ-*N*-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPEG, GRPEG, ERPEG, RRPEG, GGPRG, EGPRG, RGPRG, GEPRG, EEPRG, REPRG, GRPRG, ERPRG und RRPRG, steht, mit der Maßgabe, daß es sich bei höchstens einer R¹-Gruppierung nicht um Wasserstoff handelt.

6. Verbindungen nach Anspruch 1, wobei R¹ ausgewählt ist aus Wasserstoff und NHC(O)(CH₂)ₙCOOCH₃, worin n für eine ganze Zahl, ausgewählt aus dem Bereich 1 bis 8, handelt.

7. Pharmazeutische Zusammensetzung, umfassend eine wirksame Menge einer Verbindung nach einem der Ansprüche 1 bis 6, sowie ein pharmazeutisch annehmbares Hilfsmittel oder Verdünnungsmittel.

## Revendications

1. Composé conforme au diagramme structural I 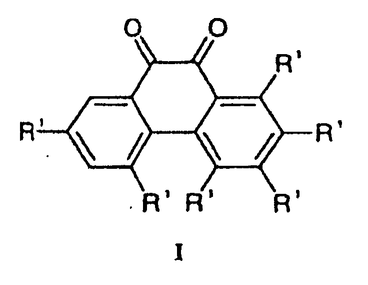 ou des tautomères de celui-ci ou des sels pharmaceutiquement acceptables de celui-ci,
dans lequel :
R¹ à chaque occurrence est choisi indépendamment parmi hydrogène, NH-CO-R², CO-NH-R², Ar, (CH₂)ₙCH(COOH)-R³, COR³, NHCOCH₂CH(COOH)NHR⁴ et N(R⁵)₂,
où
R² est choisi parmi alkyl(C₁-C₄), alkyl(C₁-C₈)COOR⁶ et phényle, où les motifs phényle de R² peuvent être substitués au niveau d'une, deux ou trois positions par un motif choisi parmi halogène, NO₂ et CF₃, les motifs alkyle de R² peuvent être substitués par halogène, et R⁶ est choisi parmi hydrogène et alkyl(C₁-C₄) ;
Ar à chaque occurrence est choisi indépendamment parmi des groupements conformes aux diagrammes structuraux suivants 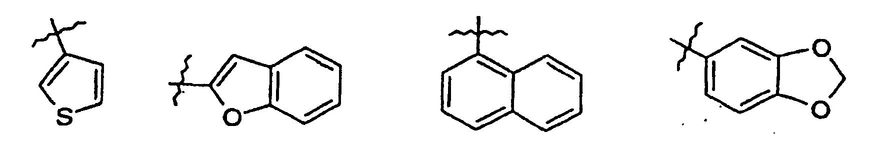 ou parmi phényle qui peut être substitué par un motif choisi parmi halogène, alkyl(C₁-C₄), O-alkyl(C₁-C₄) et halogénoalkyl(C₁-C₄) ;
R³ à chaque occurrence est un oligopeptide N-lié choisi parmi GQ-*N*-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPEG, GRPEG, ERPEG, RRPEG, GGPRG, EGPRG, RGPRG, GEPRG, EEPRG, REPRG, GRPRG, ERPRG et RRPRG,
R⁴ à chaque occurrence est un N-acétyloligopeptide C-lié choisi parmi Q, EGQ, ATEGQ, TATEGQ et FTATEGQ, et
R⁵ à chaque occurrence est choisi parmi hydrogène et tosyle ;
à condition que ledit composé ne soit pas l'acide 9,10-dioxo-9,10-dihydrophénanthrène-2-carboxylique ; l'acide 9,10-dioxo-9,10-dihydrophénanthrène-3-carboxylique ; la 3-acétylphénanthrène-9,10-dione ; la 2-acétylphénanthrène-9,10-dione ; ou la 9,10-phénanthrènedione. -

2. Composé selon la revendication 1, conforme au diagramme structural II 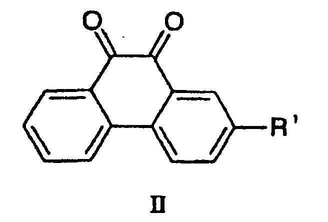 dans lequel :
R¹ est N(R⁵)₂, où R⁵ est choisi parmi hydrogène et tosyle, à condition qu'au moins un motif R⁵ soit tosyle.

3. Composé selon la revendication 1, conforme au diagramme structural III 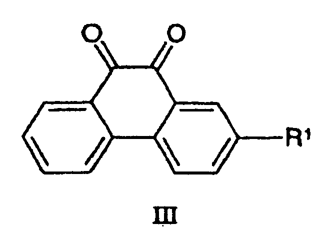 dans lequel :
R¹ est Ar et Ar est choisi parmi des groupements conformes aux diagrammes structuraux suivants 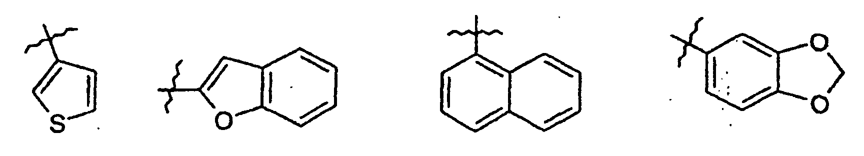
ou parmi phényle qui peut être substitué par un motif choisi parmi halogène, alkyl(C₁-C₄), O-alkyl(C₁-C₄) et halogénoalkyl(C₁-C₄).

4. Composé selon la revendication 3, dans lequel Ar est phényle substitué par perfluorométhyle.

5. Composé selon la revendication 1, conforme au diagramme structural IV 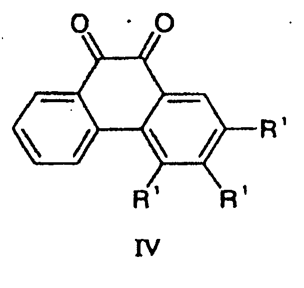 dans lequel :
R¹ est choisi parmi hydrogène, (CH₂)ₙCH(COOH)R³ et COR³, où R³ est un peptide N-lié choisi parmi GQ-*N*-iBu, GQPQP, QQPQP, EQPQP, GEPQP, QEPQP, GQGQP, QQGQP, EQGQP, GEGQP, QEGQP, QQPEG, GQGEP, GQPQG, QQPQG, GEPQG, GQPEG, GGPEG, EGPEG, RGPEG, GEPEG, EEPEG, REPEG, GRPEG, ERPEG, RRPEG, GGPRG, EGPRG, RGPRG, GEPRG, EEPRG, REPRG, GRPRG, ERPRG et RRPRG, à condition qu'un motif R¹ au plus soit autre qu'hydrogène.

6. Composé selon la revendication 1, dans lequel R¹ est choisi parmi hydrogène et NHC(O)(CH₂)ₙCOOCH₃, où n est un nombre entier choisi dans la plage de 1 à 8.

7. Composition pharmaceutique comprenant une quantité efficace d'un composé selon l'une quelconque des revendications 1 à 6, et un excipient ou diluant pharmaceutiquement acceptable.
